# EUROPEAN PATENT APPLICATION

(11) **EP 4 230 288 A2**
(43) Date of publication of application: **23.08.2023**
(21) Application number: 23178106.3
(22) Date of filing: 24.10.2012
(51) Int. Cl.: B01J 19/00, C12N 15/10, C40B 40/08, C40B 50/14, C40B 60/12, G01N 33/543, G01N 33/68

(54) **PROGRAMMABLE ARRAYS**

(30) Priority: 25.10.2011 US 201161551128 P
(62) Divisional of application: 12787975.7
(71) Applicant: Arizona Board of Regents, a Body Corporate of the State of Arizona acting for and on behalf of Arizona State University, Scottsdale, Arizona 85257-3538 (US); Engineering Arts LLC, Tempe AZ 85281 (US)
(72) Inventor: WIKTOR, Peter, Phoenix, 85044 (US); LABAER, Joshua, Chandler, 85249 (US); KAHN, Peter, Phoenix, 85048 (US); TAKULAPALLI, Bharath, Tempe, 85282 (US); QIU, Ji, Chandler, 85248 (US); BRUNNER, All, Phoenix, 85245 (US); MAGEE, Mitch, Chandler, 85224 (US)
(74) Representative: Zwicker, Jörk

(57) **Abstract**

Biomolecule arrays on a substrate are described which contain a plurality of biomolecules, such as coding nucleic acids and/or isolated polypeptides, at a plurality of discrete, isolated, locations. The arrays can be used, for example, in high throughput genomics and proteomics for specific uses including, but not limited molecular diagnostics for early detection, diagnosis, treatment, prognosis, monitoring clinical response, and protein crystallography.

## Description

### Cross reference

This application claims priority to U.S. Provisional Patent Application Serial No. 61/551,128 filed October 25, 2011, incorporated by reference herein in its entirety.

### Statement of Government Rights

This invention was made with government support under grant number R42 RR031446 awarded by National Institutes of Health. The government has certain rights in the invention.

### Background

Microarrays have revolutionized molecular biology by enabling thousands of experiments to be performed simultaneously within the size of a single microscope slide. Originally microarrays consist of thousands of spots of short nucleotide polymers that are either synthesized directly onto the microarray surface or pre-synthesized and then spotted onto the surface. These 'oligonucleotide' microarrays are typically used to detect mRNAs that correspond to gene expression. The field of microarrays has expanded to now include arrays of various different types of biological molecules (biomolecules) such as peptides, siRNA, microRNA, antibodies, or proteins. However many of these emerging microarrays have yet to reach their full potential, as research or clinical diagnostic tools, since they are more difficult to manufacture than oligonucleotide microarrays. For example, currently protein microarrays are typically manufactured by expressing and purifying thousands of proteins, which are then stored until they are printed using pin-spotters, a process flow with many inherent logistical problems. Furthermore, many proteins are unstable so these steps must all be maintained at cold temperature. Nucleic Acid Programmable Protein Arrays (NAPPA) is a well-established method that gets around these problems. It involves first printing DNA microarrays and then transcribing and translating the DNA into proteins directly on the microarray surface. This has many advantages since the DNA arrays are relatively stable, even at room temperature, and the proteins are often expressed immediately before an experiment so they remain functional. Currently NAPPA array density is limited to several thousand proteins per array. There are many compelling needs to cost-effectively manufacture higher density protein and other types of microarrays.

### Summary of the Invention

In a first aspect, the present invention provides biomolecule arrays, comprising
(a) a first substrate; and (b) biomolecules comprising at least 10 isolated coding nucleic acids and/or at least 10 isolated polypeptides, wherein each nucleic acid and/or polypeptide is physically confined at a discrete location on the first substrate, and wherein each nucleic acid is capable of expressing its encoded product in situ at its discrete location on the substrate, and/or wherein each polypeptide is capable of a characteristic activity in situ at its discrete location on the substrate; wherein the discrete locations are separated from each other on the first substrate by a center to center spacing of between about 20 nm and about 1 mm.

In a second aspect, the present invention provides arrays comprising (a) a silicon-containing substrate, having a surface comprising a plurality of nanowells at discrete locations wherein the discrete locations are separated from each other on the silicon-containing substrate by a center to center spacing of between about 20 nm and about 1 mm; and (b) biomolecules capable of capturing another molecule located at each discrete location. In one embodiment, the silicon-containing substrate is a silica or silicon substrate, such as a glass or a silicon wafer

In one embodiment of either of these aspects, the discrete locations are separated from each other on the first substrate by a center to center spacing of between about 20 nm and about 100 µm. In another embodiment, each physically confined discrete location comprises a well. In a further embodiment, each well has a diameter of between about 14 nm and about 0.75 mm. In another embodiment, the wells are present on the first substrate at a density of between about 250 billion wells per square centimeter and about 100 well per square centimeter. In a still further embodiment, the discrete locations are functionalized. In another embodiment, each discrete location further comprises reagents for expressing the encoded nucleic acid product and/or for testing polypeptide activity. In a further embodiment, each discrete location further comprises reagents for expressing the encoded nucleic acid product and/or for testing polypeptide activity and where those reagents are added to the array separately from the nucleic acids or polypeptides. In one embodiment, the reagent comprises a reticulocyte lysate. In a further embodiment, the biomolecules comprise at least 10 isolated coding nucleic acids. In another embodiment, the biomolecules comprise at least 10 isolated polypeptides. In a further embodiment, the nucleic acids and/or polypeptides are bound to the substrate.

In another embodiment of either of these aspects, either (i) the array further comprises a capture substrate adapted to mate with the first substrate wherein the capture substrate is functionalized to capture and isolate the expression product; or (ii) the discrete locations are further functionalized to capture and isolate the expression product.

In another embodiment of either of these aspects, the biomolecules comprise antigen or antibodies, and the like. In a further embodiment, the biomolecules are attached to the surface of the first substrate through a divalent linking group to a functional group capable of binding to or associating with the surface of the substrate.

In a third aspect, the present invention provides methods for in situ nucleic acid expression, comprising (a) contacting each discrete location of the array of any embodiment or combination of embodiments of the invention with reagents for nucleic acid expression to form a mixture; and (b) incubating the mixture under conditions suitable for nucleic acid expression. In a fourth aspect, the present invention provides methods for expressing and capturing a product, comprising (a) contacting a nucleic acid array with reagents for nucleic acid expression to form a mixture, wherein the nucleic acid array comprises a first substrate and at least 10 isolated coding nucleic acids physically confined at a discrete location on a first substrate, and wherein each nucleic acid is capable of expressing its encoded product in situ at its discrete location on the first substrate; (b) optionally, bringing the first substrate into the proximity of a capture substrate; (c) incubating the mixture under conditions suitable for production of nucleic acid expression products; and (d) capturing and isolating the expression products on the first substrate or, when present, the capture substrate. In one embodiment, the capture substrate comprises a second substrate that comprises a plurality of physically confined discrete locations that match the physically confined discrete locations on the first substrate. In another embodiment, the methods comprise pre-coating the first substrate or the capture substrate, when present, with a first member of a binding pair, wherein the expression products comprise a second member of the binding pair, and wherein the incubating is done under conditions suitable for binding of the first member and the second member of the binding pair, resulting in capture of the expression product on the pre-coated substrate. In a further embodiment, the methods comprise pre-coating the first substrate or the capture substrate with a first member of a binding pair. In a further embodiment, the capture substrate is contacted to the first substrate to form a seal. In another embodiment, each discrete location comprises a well. In further embodiments, the nucleic acid expression comprises RNA expression and/or protein expression. In another embodiment, the reagents comprise an in vitro expression system. In a further embodiment, the methods comprise modifying the polypeptide capture on the capture substrate. In a still further embodiment, the methods comprise further stabilizing the captured polypeptides on the capture substrate to retain protein functionality, such as by freezing the captured polypeptides on the capture substrate.

### Description of the Figures

**Figure 1** shows a side cut-through of an exemplary array substrate and an exemplary process for preparing and using the same.
**Figure 2** illustrates an exemplary method for filling and sealing nanowells on a substrate for product expression.
**Figure 3** shows the substrate of Figure 2 having a sealed cover.
**Figure 4** illustrates an exemplary method for filling nanowells on a substrate described herein.
**Figure 5** shows two separate results of a control experiment on glass, with four drops of DNA & reagents dispensed in the center spot, proteins produced from the DNA using reticulocyte lysate and the proteins tagged with fluorescently labeled antibodies.
**Figure 6** shows the same experiment as Figure 5 using silicon nanowells instead of glass with 4 drops of DNA & reagents dispensed in the center well and reticulocyte lysate sealed within each nanowell.
**Figure 7** shows the same experiment as Figure 6 with 8 drops of DNA & reagents dispensed in the center well.
**Figure 8** shows the same experiment as Figure 6 with 16 drops of DNA & reagents dispensed in the center well.
**Figure 9** shows the same experiment as Figure 6 with 32 drops of DNA & reagents dispensed in the center well.
**Figure 10** shows the same experiment as Figure 9 with reticulocyte lysate not sealed within each nanowell.
**Figure 11** illustrates an exemplary process for expressing a product within a microcapillary array.
**Figure 12** illustrates an exemplary method for detecting the presence of fluorescent molecules within and on the surface of microcapillary tubes.
**Figure 13** is an image produced according to the method illustrated in Figure 12.
Figure 14 is an image produced according to the method illustrated in Figure 12.
**Figure 15** illustrates a method for producing a protein array from a DNA array by first printing a DNA array onto a surface, filling an array of microcapillaries with reticulocyte lysate, aligning the array of microcapillaries with the DNA array, bringing the array of microcapillaries into contact with the DNA array, sealing the array of microcapillaries and then incubating the assembly to produce proteins from the DNA.
**Figure 16** illustrates an exemplary method of capture of an expression product on a secondary (capture) surface using a substrate comprising nanowells.
**Figure 17** illustrates an exemplary method of capture of an expression product on a secondary (capture) surface using a substrate comprising microcapillaries.
**Figure 18** illustrates an exemplary method for releasing a captured expression product from a substrate comprising microcapillaries.
**Figure 19** is a continuation of Figure 18, showing methods for preparing arrays or capturing solutions of the released expression products.
**Figure 20** is a continuation of Figure 18, showing exemplary diagnostic methods using the released expression products.
**Figure 21** is a continuation of Figure 18, showing other exemplary diagnostic methods using the released expression products.
**Figure 22** is a continuation of Figure 21, showing exemplary diagnostic methods using the released expression products.
**Figure 23** illustrates an exemplary method for capturing an expression product within the same nanowell as product expression.

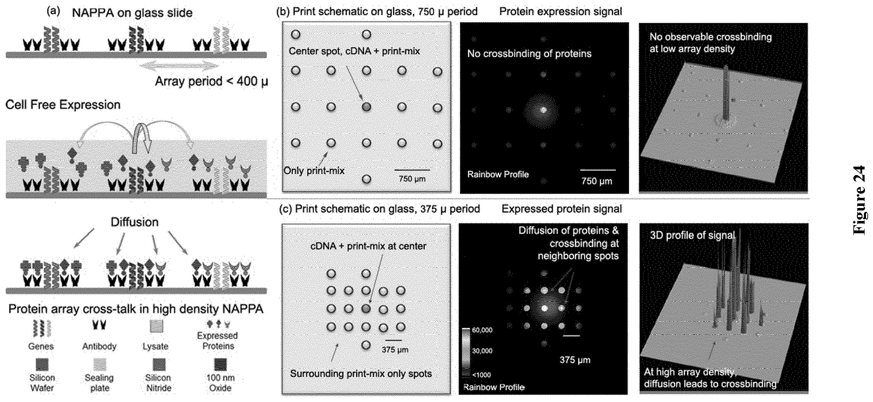

**Figure 24** illustrates diffusion on glass slides for NAPPA at high array densities. (a) Schematic of NAPPA on glass, with array spacing less than 400 microns. In-situ expressed proteins diffuse in the lysate mixture and cross-bind at neighboring locations. As shown in the print layout schematic to the left, for both (b) and (c), only the center spot was printed with DNA + printing-mix, while the surrounding spots were printed with just printing-mix consisting of anti-GST capture antibodies (no DNA). (b) NAPPA on glass slides with feature period of 750 microns, showing no observable diffusion. (c) NAPPA on glass slides with feature period of 375 microns, showing visible diffusion.

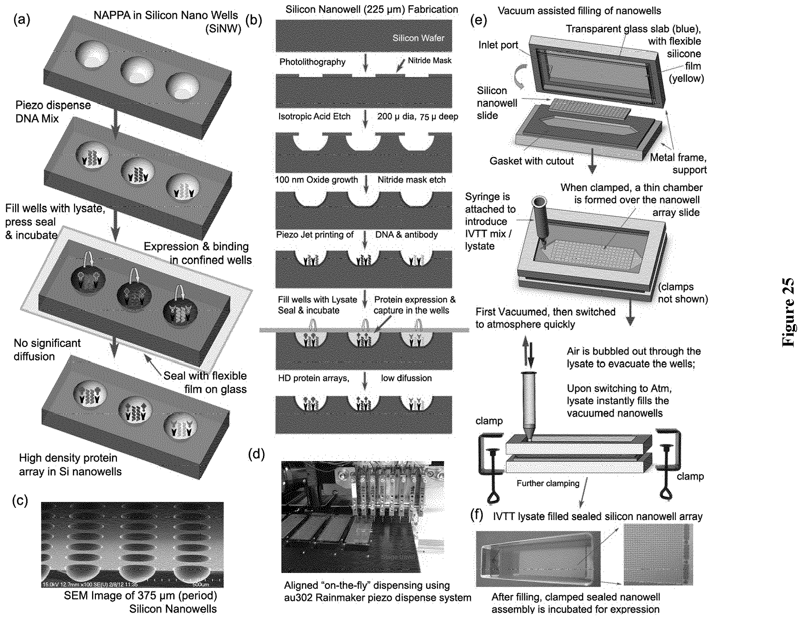

Figure 25 (a) Schematic of NAPPA in silicon nanowells; NAPPA samples were piezo dispensed in the wells, which were then filled with lysate and press-sealed with a compliant gasket film supported on a glass slab. Protein expression and subsequent capture by substrate-bound antibody occurred in confined nano-liter volumes, resulting in diffusion-free high density protein arrays (b) Method of fabrication of silicon nanowells; surface functionalization, printing and NAPPA expression (c) Cross-sectional SEM image of nanowells with 375 micron spacing (d) Engineering Arts au302 8-head piezo printer, dispensing on-the-fly into silicon nanowells (e) Schematic of vacuum assisted filling mechanism developed in-house to effectively fill silicon nanowells with IVTT lysate. Silicon nanowell slide is placed in the gasket cutout and sandwiched between the two frames. When the assembly is clamped a thin microfluidic chamber is formed over the slide, enabling filling and sealing proteins (f) Picture showing sealed nanowells filled with lysate.

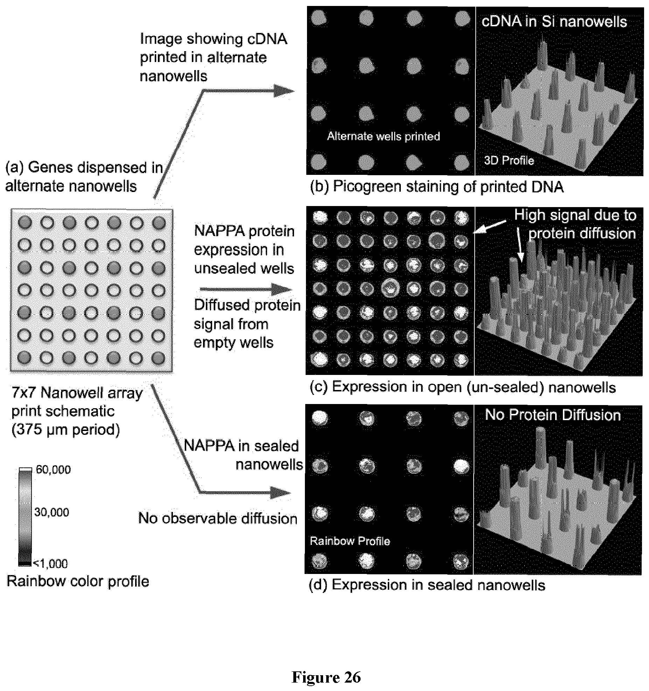

**Figure 26** illustrates confined protein expression in sealed nanowells. (a) Schematic of 16 different genes printed into alternate wells, in a 7 x 7 nanowell array (375 µm period) (b) Pico-green staining of printed DNA; to the right - 3D profile of the signal showing intensity plotted against x & y coordinates (c) Expression in unsealed nanowells detected using an anti-GST antibody. Expressed proteins diffuse locally and physically-adsorb inside neighboring wells, displaying strong signal in all the wells (d) Expression in sealed nanowells detected using an anti-GST antibody. The empty wells in-between do not show any signal, implying no diffusion of protein from sealed wells. 3D profile of protein display to the right clearly shows diffusion-free signals in sealed nanowells. (Refer to Supporting Information for cDNA print details).

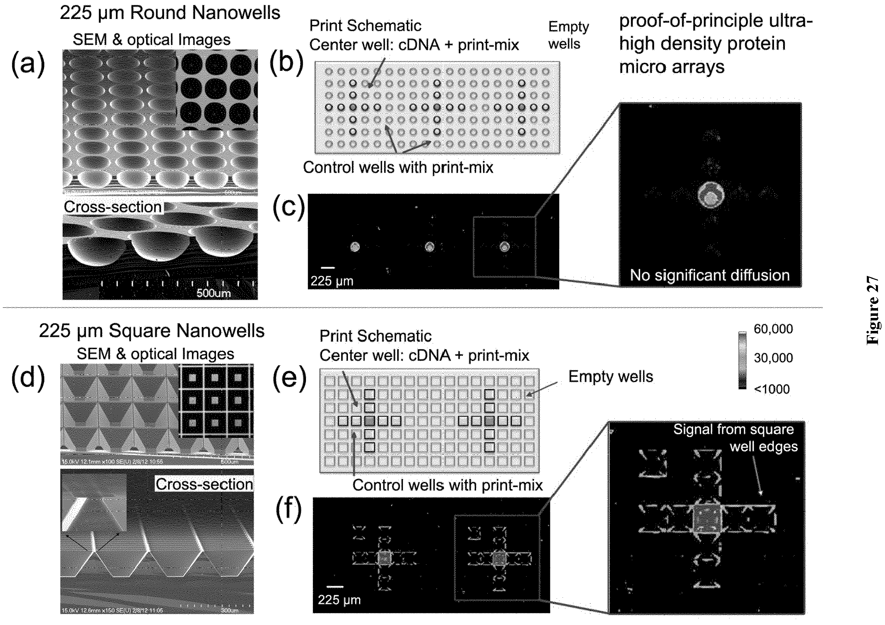

**Figure 27** Demonstration of very high density protein arrays towards 24,000 proteins on a single slide. 225 micron period nanowell arrays were produced in round-well and square-well geometries, by using different silicon wet-etch chemistries. In both cases, control printing-mix spots (no cDNA) were printed in a plus pattern around the central expression spots. Neither the control printing-mix spots comprising antibodies nor the surrounding empty spots show significant protein diffusion signal (a) Scanning electron microscope (SEM) images of 225 micron period round silicon wells in top and cross-sectional views; inset shows optical microscope image (b) Schematic of print layout in 225 micron period round nanowell array (c) Corresponding protein array display showing high intensity from center cDNA spots with no significant diffusion background (d) SEM and optical microscope images of square nanowell arrays in top and cross-sectional views (e) Schematic of print layout in 225 micron period square nanowell array (f) Expressed proteins are displayed with strong signals (square shaped) from cDNA printed nanowells, while printing-mix printed wells show signals at the sharp edges of the well (and empty wells show no discernible signal). Signal from square edges is thought to be due to preferential aggregation of proteins and dye at the sharp edges of square wells.

### Detailed Description of the Invention

All references cited are herein incorporated by reference in their entirety. Within this application, unless otherwise stated, the techniques utilized may be found in any of several well-known references such as: Molecular Cloning: A Laboratory Manual (Sambrook, et al., 1989, Cold Spring Harbor Laboratory Press), Gene Expression Technology (Methods in Enzymology, Vol. 185, edited by D. Goeddel, 1991. Academic Press, San Diego, CA), "Guide to Protein Purification" in Methods in Enzymology (M.P. Deutshcer, ed., (1990) Academic Press, Inc.); PCR Protocols: A Guide to Methods and Applications (Innis, et al. 1990. Academic Press, San Diego, CA), Culture of Animal Cells: A Manual of Basic Technique, 2nd Ed. (R.I. Freshney. 1987. Liss, Inc. New York, NY), Gene Transfer and Expression Protocols, pp. 109-128, ed. E.J. Murray, The Humana Press Inc., Clifton, N.J.), and the Ambion 1998 Catalog (Ambion, Austin, TX).

All embodiments disclosed herein can be combined with other embodiments unless the context clearly dictates otherwise.

Unless the context clearly requires otherwise, throughout the description and the claims, the words "comprise," "comprising," and the like are to be construed in an inclusive sense as opposed to an exclusive or exhaustive sense; that is to say, in the sense of "including, but not limited to." Words using the singular or plural number also include the plural or singular number, respectively. Additionally, the words "herein," "above," and "below" and words of similar import, when used in this application, shall refer to this application as a whole and not to any particular portions of this application. As used herein, the singular forms "a", "an" and "the" include plural referents unless the context clearly dictates otherwise. "And" as used herein is interchangeably used with "or" unless expressly stated otherwise.

As used herein, the term "about" means within 5% of the recited limitation.

The description of embodiments of the disclosure is not intended to be exhaustive or to limit the disclosure to the precise form disclosed. While specific embodiments of, and examples for, the disclosure are described herein for illustrative purposes, various equivalent modifications are possible within the scope of the disclosure, as those skilled in the relevant art will recognize. For example, while method steps or functions are presented in a given order, alternative embodiments may perform functions in a different order, or functions may be performed substantially concurrently. The teachings of the disclosure provided herein can be applied to other procedures or methods as appropriate. The various embodiments described herein can be combined to provide further embodiments.

In one aspect, the disclosure provides biomolecule arrays, comprising (a) a first substrate; and (b) biomolecules comprising at least 10 isolated coding nucleic acids and/or at least 10 isolated polypeptides, wherein each nucleic acid and/or polypeptide is physically confined at a discrete location on the first substrate, and wherein each nucleic acid is capable of expressing its encoded product in situ at its discrete location on the substrate, and/or wherein each polypeptide is capable of a characteristic activity in situ at its discrete location on the substrate; wherein the discrete locations are separated from each other on the first substrate by a center to center spacing of between about 20 nm and about 1 mm.

The arrays of the invention can be used, for example, in high throughput genomics and proteomics for specific uses including, but not limited molecular diagnostics for early detection, diagnosis, treatment, prognosis, monitoring clinical response, and protein crystallography.

Biomolecules of the present arrays can be a nucleic acid or a polypeptide that is capable of producing an expression product when incubated with appropriate reagents (enzymes, buffers, salts, nucleotides, amino acids, ribosomes, etc.), or of being tested for a specific activity on the substrate.

When the biomolecule is a polypeptide, such polypeptides may comprise or consist of full length proteins, protein fragments, and naturally occurring and synthetic peptides. The polypeptides may include non-naturally occurring amino acids and other modifications as desired for a given purpose.

When the biomolecule is a nucleic acid, the nucleic acid can be RNA or DNA. (e.g., a single-stranded DNA, or a double stranded DNA). In a preferred embodiment, the nucleic acid includes a plasmid or viral DNA or a fragment thereof; an amplification product (e.g., a product generated by RCA, PCR, NASBA); or a synthetic DNA. The nucleic acid may further include one or more of: a transcription promoter; a transcription regulatory sequence; a untranslated leader sequence; a sequence encoding a cleavage site; a recombination site; a 3' untranslated sequence; a transcriptional terminator; a sequence encoding an epitope tag; and an internal ribosome entry site. In another embodiment, the nucleic acid also includes a sequence encoding a reporter protein, e.g., a protein whose abundance can be quantitated and can provide an indication of the quantity of expression product. The reporter protein can be attached to the nucleic acid expression product, e.g., covalently attached, e.g., attached as a translational fusion. The reporter protein can be an enzyme, e.g., β-galactosidase, chloramphenicol acetyl transferase, β-glucuronidase, and so forth. The reporter protein can produce or modulate light, e.g., a fluorescent protein (e.g., green fluorescent protein, variants thereof, red fluorescent protein, variants thereof, and the like), and luciferase. The transcription promoter can be a prokaryotic promoter, a eukaryotic promoter, or a viral promoter. The regulatory components, e.g., the transcription promoter, can vary among nucleic acids at different addresses of the plurality. For example, different promoters can be used to vary the amount of polypeptide produced at different addresses.

The biomolecules may be bound to the substrate, or may be unbound. Methods for binding biomolecules to substrates are well known in the art, as described below. The biomolecules at each discrete position can all be the same, or can vary from one position to another, as desired for any given purpose. The expression products may be the same at each location, may differ at each location, or any other configuration, as desirable for a given purpose.

As used herein, the term "substrate" refers to any type of solid support, such as the silicon-containing substrates described below, or any of the following substrates on which the biomolecules can be arrayed. Examples of such substrates include, but are not limited to, microarrays, beads, columns, optical fibers, wipes, nitrocellulose, nylon, glass, quartz, diazotized membranes (paper or nylon), silicones, polyformaldehyde, cellulose, cellulose acetate, paper, ceramics, metals, metalloids, semiconductive materials, coated beads, magnetic particles; plastics such as polyethylene, polypropylene, and polystyrene; and gel-forming materials, such as proteins (e.g., gelatins), lipopolysaccharides, silicates, agarose, polyacrylamides, methylmethracrylate polymers; sol gels; porous polymer hydrogels; nanostructured surfaces; nanotubes (such as carbon nanotubes, self-assembling-monolayers of functionalized molecules and nanoparticles (such as gold nanoparticles or quantum dots).

In other embodiments, the first substrate may be a thermoplastic molded substrate via microtransfer molding or hot embossing from a suitable mold. The mold can be a patterned PDMS, silicon, or metal (e.g., Ni) mold prepared by methods familiar to those skilled in the art.

In one exemplary embodiment, the substrate comprises a substrate suitable for use in a "dipstick" device, such as one or more of the substrates disclosed above. When bound to a substrate, the biomolecule can be directly linked to the support, or attached to the surface via a linker. Thus, the substrate and/or the biomolecules can be derivatized using methods known in the art to facilitate binding of the biomolecules to the support. Other molecules, such as reference or control molecules, can be optionally immobilized on the surface as well. Methods for immobilizing various types of molecules on a variety of surfaces are well known to those of skill in the art. A wide variety of materials can be used for the functionalized solid support surface including, but not limited to, glass, gold or silicon for example.

The biomolecule is physically confined at a discrete location, such that the biomolecule is separated from biomolecules at other discrete locations by any suitable type of barrier. In various non-limiting embodiments, the discrete location can be a well, a tube, a patterned (chemical or mechanical) region of the substrate, a pillar (in relief), or any three-dimensional feature(s) that inhibit lateral diffusion between adjacent locations.

In one embodiment, the arrays and methods disclosed herein provide dramatic improvements of protein microarrays; particularly to reduce manufacturing costs, improve quality and increase the density of Nucleic Acid Programmable Arrays (NAPPA). NAPPA is a means of in situ expression and capture of thousands of different proteins in a microarray format. In this method, DNA molecules corresponding to the proteins of interest are printed on the microarray substrate and then transcribed/translated in situ at the time of assay. The DNA molecules are configured to append a common epitope tag to all of the proteins on the N- or C-termini so that they can be captured by a high-affinity capture reagent that is immobilized along with the DNA and bovine serum albumen (BSA) and BS3 cross-linker. In vitro transcription and translation (IVTT)-coupled reagents, such as rabbit reticulocyte lysate, are used to produce the protein. The expressed protein is captured on the array through the high affinity reagent that recognizes the epitope tag. NAPPA microarrays are used by researchers to concurrently study the interactions of thousands of different proteins on the microarray surface with another biomolecule, drug candidate or serum sample.

Physical confinement of the discrete locations prevents diffusion during, for example, in vitro protein expression of NAPPA. In certain embodiments, each of the physically confined discrete locations can comprise a well. As used herein, a well is a fluid receptacle that is open at one end and closed at the other end. The wells can be of any desired shape, including but not limited to circular, rectangular, square, polygonal, or arbitrarily shaped. Wells can be made by any suitable technique, including but not limited to: photolithography and/or isotropic or anisotropic etch in silicon wafers; forming a silicon-dioxide layer on top of the silicon wafer for compatibility with NAPPA chemistry that has been developed for glass (silicon-dioxide) surfaces; forming the nanowells by micro/Nano-imprinting of PDMS or by photolithography of SU8; etching of glass or bonding a perforated membrane to a solid surface. In one embodiment, the substrates comprise one or more further features to improve sealing of wells during biochemical processing, when stored or in use. Sealing prevents molecules from diffusing from one well to the next during a biochemical processes, and is especially useful for high-density arrays with small well-to-well spacing. Such features include, but are not limited to, a mating substrate that serves to seal each well; a lip around each well (where the lip comprises the same or different material as the substrate is made of), a silicone-rubber gasket around each well, and a mating substrate that forms a seal around each well of the substrate. For example, the mating substrate may have a compliant material to form a tight seal between discrete locations when it is mated with the substrate.

In certain embodiments, each well has a diameter of between about 14 nm and about 0.75 mm. In various embodiments, the well diameters are between 20 nm and 500 µm; 50 nm and 500 µm; 100 nm and 500 µm; 250 nm and 500 µm; 500 nm and 500 µm; 1 µm and 500 µm; 10 µm and 500 µm; 100 µm and 500 µm; 150 µm and 500 µm; 20 nm and 300 µm; 50 nm and 300 µm; 100 nm and 300 µm; 250 nm and 300 µm; 500 nm and 300 µm; 1 µm and 300 µm; 10 µm and 300 µm; 100 µm and 300 µm; 150 µm and 300 µm; 20 nm and 250 µm; 50 nm and 250 µm; 100 nm and 250 µm; 250 nm and 250 µm; 500 nm and 250 µm; 1 µm and 250 µm; 10 µm and 250 µm; 100 µm and 250 µm; and 150 µm and 250 µm.

The wells can be present on the first substrate at a density of between about 250 billion wells per square centimeter and about 100 well per square centimeter. In various embodiments, the wells are present on the first substrate at a density of between about 250 billion wells per square centimeter and about 500 wells per square centimeter; about 250 billion wells per square centimeter and about 1000 wells per square centimeter; about 250 billion wells per square centimeter and about 10,000 wells per square centimeter; about 100 billion wells per square centimeter and about 100 wells per square centimeter; about 100 billion wells per square centimeter and about 500 wells per square centimeter; about 100 billion wells per square centimeter and about 1000 wells per square centimeter; about 100 billion wells per square centimeter and about 10,000 wells per square centimeter; about 10 billion wells per square centimeter and about 100 wells per square centimeter; about 10 billion wells per square centimeter and about 500 wells per square centimeter; about 10 billion wells per square centimeter and about 1000 wells per square centimeter; about 10 billion wells per square centimeter and about 10.000 wells per square centimeter; about 1 billion wells per square centimeter and about 100 wells per square centimeter; about 1 billion wells per square centimeter and about 500 wells per square centimeter; about 1 billion wells per square centimeter and about 1000 wells per square centimeter; about 1 billion wells per square centimeter and about 10,000 wells per square centimeter; about 100 million wells per square centimeter and about 100 wells per square centimeter; about 100 million wells per square centimeter and about 500 wells per square centimeter; about 100 million wells per square centimeter and about 1000 wells per square centimeter; about 100 million wells per square centimeter and about 10,000 wells per square centimeter; about 10 million wells per square centimeter and about 100 wells per square centimeter; about 10 million wells per square centimeter and about 500 wells per square centimeter; about 10 million wells per square centimeter and about 1000 wells per square centimeter; about 10 million wells per square centimeter and about 10.000 wells per square centimeter; about 1 million wells per square centimeter and about 100 wells per square centimeter; about 1 million wells per square centimeter and about 500 wells per square centimeter; about 1 million wells per square centimeter and about 1000 wells per square centimeter; about 1 million wells per square centimeter and about 10,000 wells per square centimeter; about 100,000 wells per square centimeter and about 100 wells per square centimeter; about 100,000 wells per square centimeter and about 500 wells per square centimeter; about 100,000 wells per square centimeter and about 1000 wells per square centimeter; about 100,000 wells per square centimeter and about 10,000 wells per square centimeter; about 75,000 wells per square centimeter and about 100 wells per square centimeter; about 75,000 wells per square centimeter and about 500 wells per square centimeter; about 75,000 wells per square centimeter and about 1000 wells per square centimeter; about 75,000 wells per square centimeter and about 10,000 wells per square centimeter; about 50,000 wells per square centimeter and about 100 wells per square centimeter; about 50,000 wells per square centimeter and about 500 wells per square centimeter; about 50,000 wells per square centimeter and about 1000 wells per square centimeter; and about 50,000 wells per square centimeter and about 10,000 wells per square centimeter.

In various embodiments, the wells have a depth of between about 10 µm and about 150 µm; about 10 µm and about 125 µm; about 10 µm and about 100 µm; about 10 µm and about 90 µm; about 10 µm and about 80 µm; about 10 µm and about 75 µm; about 10 µm and about 70 µm; about 10 µm and about 60 µm; about 10 µm and about 50 µm; 25 µm and about 100 µm; about 25 µm and about 150 µm; about 25 µm and about 125 µm; about 25 µm and about 100 µm; about 25 µm and about 90 µm; about 25 µm and about 80 µm; about 25 µm and about 75 µm; about 25 µm and about 70 µm; about 25 µm and about 60 µm; about 25 µm and about 50 µm; about 50 µm and about 150 µm; about 50 µm and about 125 µm; about 50 µm and about 100 um; about 50 µm and about 90 µm; and about 50 µm and about 75 µm.

In various embodiments, the wells have a period (i.e.: spacing) on the array of between about 100 µm and about 400 µm; about 100 µm and about 375 µm; about 100 µm and about 350 µm; about 100 µm and about 325 µm; about 100 µm and about 300 µm; about 100 µm and about 275 µm; about 100 µm and about 250 µm; about 100 µm and about 225 µm; about 100 µm and about 200 µm; about 100 µm and about 185 µm; about 100 µm and about 175 µm; about 100 µm and about 150 µm; 125 µm and about 400 µm; about 125 µm and about 375 µm; about 125 µm and about 350 µm; about 125 µm and about 325 µm; about 125 µm and about 300 µm; about 125 µm and about 275 µm; about 125 µm and about 250 µm; about 125 µm and about 225 µm; about 125 µm and about 200 µm; about 125 µm and about 185 µm; about 125 µm and about 175 µm; about 125 µm and about 150 µm; 150 µm and about 400 µm; about 150 µm and about 375 µm; about 150 µm and about 350 µm; about 150 µm and about 325 µm; about 150 µm and about 300 µm; about 150 µm and about 275 µm; about 150 µm and about 250 µm; about 150 µm and about 225 µm; about 150 µm and about 200 µm; about 150 µm and about 185 µm; about 150 µm and about 175 µm; 175 µm and about 400 µm; about 175 µm and about 375 µm; about 175 µm and about 350 µm; about 175 µm and about 325 µm; about 175 µm and about 300 µm; about 175 µm and about 275 µm; about 175 µm and about 250 µm; about 175 µm and about 225 µm; about 175 µm and about 200 µm; about 175 µm and about 185 µm; about 185 µm and about 400 µm; about 185 µm and about 375 µm; about 185 µm and about 350 µm; about 185 µm and about 325 µm; about 185 µm and about 300 µm; about 185 µm and about 275 µm; about 185 µm and about 250 µm; about 185 µm and about 225 µm; and about 185 µm and about 200 µm.

In another embodiment, where well spacing (in mm) is "Sp", density is "Dn", diameter is "Dm", and depth is "Dp".
(a) the well density (in spots/mm^2) is 1/Sp^2 <= Dn <= 1/(Sp^{∗}(0.75)^{0.5})²;
(b) the well diameter (in mm) is 0.1 *Sp <= Dm <= 0.95*Sp; and/or
(c) the well depth is (in mm) is 0.1 *Dm <= Dp <= 3*Dm; and

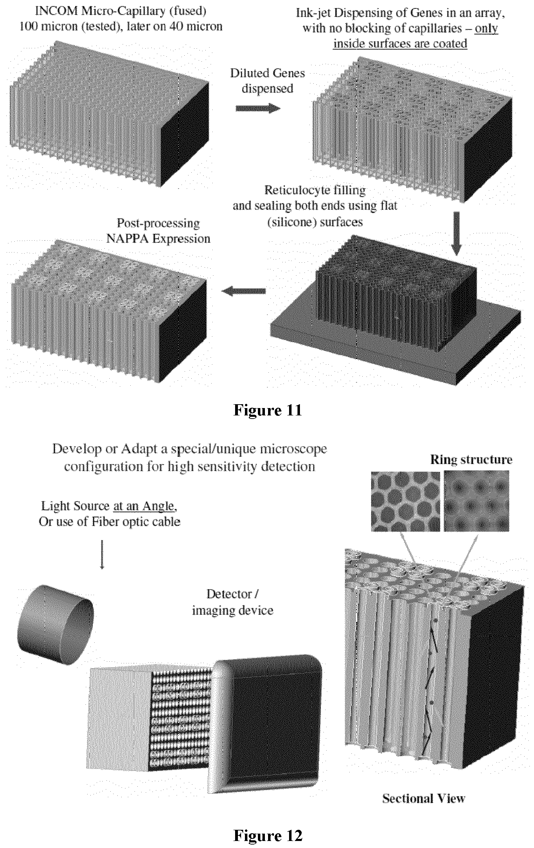

In other embodiments, each physically confined discrete location comprises a tube **(****Figure 11****).** As used herein, a tube is a fluid receptacle that is open at both ends. The "tube" may be one or more (i.e.: 2, 3, 4, 5, 6, 7, 8, 9, 10, or more) adjacent tubes in a single location. In one preferred embodiment, a plurality of tubes in a discrete location is bundled together. In another embodiment, a plurality of tubes is fused at one end with the other ends remaining separate. As a result, each tube will, in use, receive the same fluid sample. The tubes can be of any desired shape, including but not limited to circular, rectangular, square, polygonal or arbitrarily shaped.

Tubes can be made by any suitable technique, including but not limited to: bundling glass capillaries together, fusing and drawing them out and then slicing and polishing the result into thin sheets of fused nanotubes; photolithography and/or isotropic or anisotropic etch in silicon wafers; forming a silicon-dioxide layer on the surfaces of the nanotubes for compatibility with NAPPA chemistry that has been developed for glass (silicon-dioxide) surfaces; forming the nanotubes by nano-imprinting of PDMS or by photolithography of SU8 or etching of glass.

In certain embodiments, each tube can have an inner diameter of between about 14 nm and about 0.75 mm. The tubes may be present on the first substrate at a density of between about 250 billion tubes per square centimeter and about 1 tube per square centimeter. In another embodiment the tubes are present on the first substrate at a density of between about 250 billion tubes per square centimeter and about 10 tubes per square centimeter. In another embodiment the wells are present on the first substrate at a density of between about 250 billion tubes per square centimeter and about 100 tubes per square centimeter. All embodiments of well diameter, period, and spacing on the array are equally applicable to tube inner diameter, period, and spacing. Tube depth can be any suitable depth based on length of capillary tubes used. In one embodiment, tube length is between 10 nm and 10 mm. In various further embodiments, the tube length is between 10 nm and 5 mm; 10 nm and 1 mm; 10 nm and 100 µm; 10 nm and 50 µm; 10 nm and 10 µm; 10 nm and 1 µm; 10 nm and 100 nm; 25 nm and 10 mm; 25 nm and 5 mm; 25 nm and 1 mm; 25 nm and 100 µm; 25 nm and 50 µm; 25 nm and 10 µm; 25 nm and 1 µm; 25 nm and 100 nm; 50 nm and 10 mm; 50 nm and 5 mm; 50 nm and 1 mm; 50 nm and 100 µm; 50 nm and 50 µm; 50 nm and 10 µm; 50 nm and 1 µm; 25 nm and 100 nm; 100 nm and 10 mm; 100 nm and 5 mm; 100 nm and 1 mm; 100 nm and 100 µm; 100 nm and 50 µm; 100 nm and 10 µm; 100 nm and 1 µm; 500 nm and 10 mm; 500 nm and 5 mm; 500 nm and 1 mm; 500 nm and 100 µm; 500 nm and 50 µm; 500 nm and 10 µm; 500 nm and 1 µm; 1 µm and 10 mm; 1 µm and 5 mm; 1 µm and 1 mm; 1 µm and 100 µm; 1 µm and 50 µm; 1 µm and 10 µm; 10 µm and 10 mm; 10 µm and 5 mm; 10 µm and 1 mm; 10 µm and 100 µm; 10 µm and 50 µm; 100 µm and 10 mm; 100 µm and 5 mm; and 100 µm and 1 mm.

Each of the discrete locations may be additionally functionalized. In certain embodiments, the surface is functionalized to be hydrophilic, as described below. Such hydrophilic surfaces can promote wetting and spreading of reagents. In certain embodiments, the surface is functionalized to be hydrophobic, as described below. Such surface functionalization may be within each discrete location or between the discrete locations; in the latter case, the surface functionalization may serve to define the discrete locations. In one embodiment when the substrate comprises silicon, the Si substrates (such as substrates where each of the physically confined discrete locations comprises a well) may be functionalized by coating with oxide, to reduce quenching of fluorescence by Si. In this embodiment, any suitable thickness of oxide can be coated on the substrate, such as between about 10 nm and 500 nm in thickness, preferably between about 50 nm and 250 nm in thickness, or about 100 nm in thickness.

Each discrete location can further comprise reagents for expressing the encoded nucleic acid product and/or for testing polypeptide activity. Any suitable reagents for a given purpose can be used. Those of skill in the art can determine appropriate reagents for a given use, based on the teachings herein combined with the level of skill in the art. In one non-limiting example, where the biomolecule comprises a DNA molecule and the desired expression product is a polypeptide, the reagents may comprise a reticulocyte lysate.

For example, the reagents may comprise reagents for expressing the encoded nucleic acid product and/or for testing polypeptide activity and where those reagents are added to the array separately from the nucleic acids or polypeptides. The reagents can either by added prior to adding the nucleic acids or polypeptides, or they can be added after the nucleic acids or polypeptides. For NAPPA for example, the reagents consist of a mixture of capture antibodies, BSA and BS3. This mixture can be combined with the nucleic acids prior to printing them onto the array surface or they can be printed separately. Furthermore, the nucleic acids can be printed onto the array surface with a suitable chemistry to bind them to the array surface and then the mixture can be flooded over the whole array surface. In particular embodiments, the reagent can comprise a reticulocyte lysate. The reticulocyte lysate can be a solution (for example, added by an end user), or may be placed at the discrete locations and frozen (for example, by a manufacturer), such that it can be stored, shipped, etc. Upon expression, the end products may be proteins that crystalize inside each physically confined discrete location.

In certain embodiments, the biomolecules comprise at least 10 isolated coding nucleic acids. As will be clear to those of skill in the art, more than 10 isolated coding nucleic acids and/ isolated polypeptides can be arrayed on the substrate; in various embodiments, 50, 100, 500, 1000, 2500, 5000, 10,000, 25,000, 50,000, 100,000, or more isolated coding nucleic acids and/ isolated polypeptides can be arrayed on the substrate. In other embodiments, the biomolecules comprise at least 100 isolated coding nucleic acids; or at least 1000 isolated coding nucleic acids; or at least 10 isolated polypeptides; or at least 100 isolated polypeptides; or at least 1000 isolated polypeptides. In any of the preceding embodiments, nucleic acids and/or polypeptides can be bound to the substrate.

The arrays may further comprise either (i) a capture substrate adapted to mate with the first substrate wherein the capture substrate is functionalized to capture and isolate the expression product; or (ii) the discrete locations are further functionalized to capture and isolate the expression product. As used herein, "mate" means to form a seal, such that each of the discrete locations is completely separated from each other. However, a complete seal is not necessary, proximity, on the order of micrometers, may be good enough in some cases. The capture substrate can be flat or it can have physical features that mate with those on the first substrate. In certain embodiments, the capture substrate forms a complete seal with the first substrate. The capture substrate and/or the first substrate can have a compliant material to form a tight seal between discrete locations when the two are mated together.

In another aspect, the disclosure provides an array comprising a silicon-containing substrate, having a plurality of nanowells at discrete locations formed in a surface of the substrate wherein the discrete locations are separated from each other on the silicon-containing substrate by a center to center spacing of between about 20 nm and about 1 mm; and biomolecules capable of capturing another molecule located at each discrete location. In certain embodiments, the discrete locations are separated from each other on the first substrate by a center to center spacing of between about 20 nm and about 100 µm.. All embodiments of well diameter, period, depth, and spacing on the array provided above are equally applicable in this aspect of the invention.

In one embodiment, the arrays comprise one or more further features to improve sealing of wells when stored or in use. Such features include, but are not limited to, a lip around each well (where the lip comprises the same or different material as the substrate is made of), a silicone-rubber gasket around each well, and a mating substrate that forms a seal around each well of the substrate. For example, the mating substrate may have a compliant material to form a tight seal between discrete locations when it is mated with the substrate.

Examples of biomolecules which may be located in the nanowells at each of the discrete locations include, but are not limited to, nucleic acids, antigen, antibodies, and the like, as discussed above. When the biomolecule is a polypeptide, such polypeptides may comprise or consist of full length proteins, protein fragments, and naturally occurring and synthetic peptides. The polypeptides may include non-naturally occurring amino acids and other modifications as desired for a given purpose.

When the biomolecule is a nucleic acid, the nucleic acid can be RNA or DNA. (e.g., a single-stranded DNA, or a double stranded DNA). In a preferred embodiment, the nucleic acid includes a plasmid or viral DNA or a fragment thereof; an amplification product (e.g., a product generated by RCA, PCR, NASBA); or a synthetic DNA. The nucleic acid may further include one or more of: a transcription promoter; a transcription regulatory sequence; a untranslated leader sequence; a sequence encoding a cleavage site; a recombination site; a 3' untranslated sequence; a transcriptional terminator; a sequence encoding an epitope tag; and an internal ribosome entry site. In another embodiment, the nucleic acid also includes a sequence encoding a reporter protein, e.g., a protein whose abundance can be quantitated and can provide an indication of the quantity of expression product. The reporter protein can be attached to the nucleic acid expression product, e.g., covalently attached, e.g., attached as a translational fusion. The reporter protein can be an enzyme, e.g., β-galactosidase, chloramphenicol acetyl transferase, β-glucuronidase, and so forth. The reporter protein can produce or modulate light, e.g., a fluorescent protein (e.g., green fluorescent protein, variants thereof, red fluorescent protein, variants thereof, and the like), and luciferase. The transcription promoter can be a prokaryotic promoter, a eukaryotic promoter, or a viral promoter. The regulatory components, e.g., the transcription promoter, can vary among nucleic acids at different addresses of the plurality. For example, different promoters can be used to vary the amount of polypeptide produced at different addresses.

The biomolecules may be confined at the nanowells according to methods familiar to those skilled in the art; for example, any of the preceding can be attached to the surface of the nanowells on the silicon-containing substrate through a divalent linking group to a functional group capable of binding to or associating with the surface of the substrate, as discussed below.

Examples of molecules that the biomolecules may capture include, but are not limited to, nucleic acid expression products when incubated with appropriate reagents (enzymes, buffers, salts, nucleotides, amino acids, ribosomes, etc.). In other embodiments, the biomolecules which may capture and isolate the expression product include, but are not limited to an antigen, inhibitor (e.g., an irreversible inhibitor), or an antibody for the expression product. Such surface functionalizations may be introduced by methods familiar to those skilled in the art.

In certain embodiments, the silicon-containing (Si) substrate is a silica or silicon substrate. For example, the substrate may comprise a glass or a silicon wafer (each having a shape suitable for its intended purpose). Such substrates may be chemically patterned (e.g., microcontact printing) or etched (e.g., standard masking and etching methods for silicon) according to methods known to those skilled in the art to provide a plurality of discrete locations on a surface thereof. For example, *see e.g.,* Xia and Whitesides, Ann. Rev. Mater. Sci. 1998, 28, 153, which is hereby incorporated by reference in its entirety.

In one embodiment, the Si nanowells are coated with oxide, to reduce quenching of fluorescence by Si. In this embodiment, any suitable thickness of oxide can be coated on the substrate, such as between about 10 nm and 500 nm in thickness, preferably between about 50 nm and 250 nm in thickness, or about 100 nm in thickness.

Suitable linking groups include, but are not limited to a group of the formula, -(C₀-C₁₀ alkyl-Q)₀₋₁-C₀-C₁₀ alkyl-, wherein Q is a bond, aryl, heteroaryl, C₃-C₈ cycloalkyl, or heterocyclyl; and no more than one methylene in each alkyl group is optionally and independently replaced by - O-, -S-, -N(R⁰⁰)-, -C(H)=C(H)-, -C=C-, -C(O)-, -S(O)-, -S(O)₂-, -P(O)(OH)-, -OP(O)(OH)-, -P(O )(OH)O-, -N(R⁰⁰)P(O)(OH)-, -P(O)(OH)N(R⁰⁰)-, -OP(O)(OH)O-, -OP(O)(OH)N(R⁰⁰)-, -N(R⁰⁰)P (O)(OH)O-, -N(R⁰⁰)P(O)(OH)N(R⁰⁰)-, -C(O)O-, -C(O)N(R⁰⁰)-, -OC(O)-, -N(R⁰⁰)C(O)-, -S(O)O-, -OS(O)-, -S(O)N(R⁰⁰)-, -N(R⁰⁰)S(O)-, -S(O)₂O-, -OS(O)₂-, -S(O)₂N(R⁰⁰)-, -N(R⁰⁰)S(O)₂-, OC(O)O-, -OC(O)N(R⁰⁰)-, -N(R⁰⁰)C(O)O-, -N(R⁰⁰)C(O)N(R⁰⁰)-, -OS(O)O-, -OS(O)N(R⁰⁰)-, -N( R⁰⁰)S(O)O-, -N(R⁰⁰)S(O)N(R⁰⁰)-, -OS(O)₂O-, -OS(O)₂N(R⁰⁰)-, -N(R⁰⁰)S(O)₂O-, or -N(R⁰⁰)S(O)₂N(R⁰⁰)- , wherein each R⁰⁰ is independently hydrogen or C₁-C₆ alkyl.

Suitable functional groups include, but are not limited to -NH₂ (amine), -COOH (carboxyl), siloxane (-Si(OR)₃, where each R is C₁-C₄ alkyl), -OH (hydroxyl), -SH (mercapto), - CONH₂ (amido), -P(O)(OH)₂ (phosphonic acid), -S(O)₂OH (sulfonate), -S(O)OH (sulfinate), -OS(O)₂OH (sulfate), and chemical groups including the same.

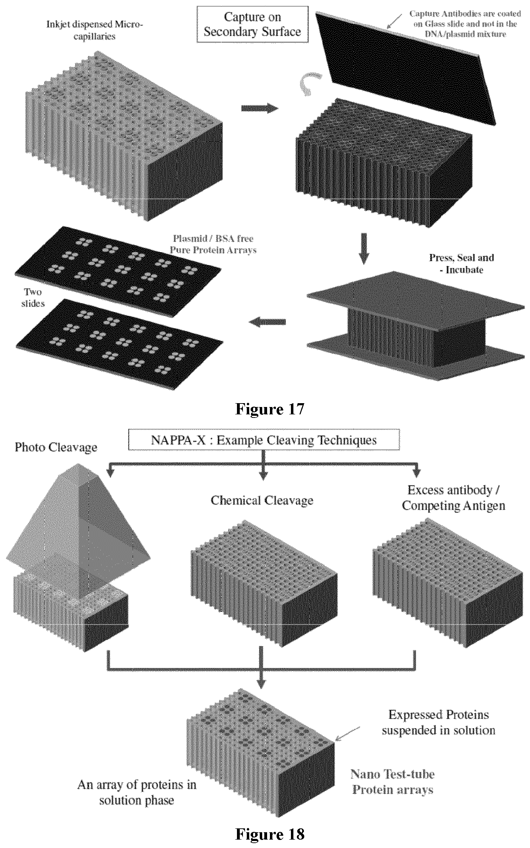

The divalent linker may also comprise a photocleavable group within the divalent group. Such surface functionalization can allow for expression products to be isolated on the surface by binding with the biomolecule capable of capturing the expression products. After expression, the substrate may be washed by methods familiar to those in the art to remove leftover starting materials, reactants, and/or contaminants. Then, the washed substrate may be exposed to a suitable wavelength of light for a period of time suitable to cleave the photocleavable linker **(****Figure 18****),** thereby releasing the expression product to allow further manipulation of the expression product (e.g., isolation on a second substrate as discussed below).

The divalent linker may also comprise a chemically cleavable group within the divalent group. Such surface functionalization can allow for expression products to be isolated on the surface by binding with the biomolecule capable of capturing the expression products. After expression, the substrate may be washed by methods familiar to those in the art to remove leftover starting materials, reactants, and/or contaminants. Then, the washed substrate may be exposed to a suitable reagent for a period of time suitable to cleave the chemically cleavable linker, thereby releasing the expression product to allow further manipulation of the expression product (e.g., isolation on a second substrate as discussed below).

In certain embodiments, the surface is functionalized to be hydrophilic. Examples of hydrophilic functionalizations include, but are not limited to hydroxyalkyl, aminoalkyl, or carboxyalkyl-functionalized siloxanes (for silica surfaces; e.g., 3-aminopropyl trimethoxysilane, (N,N-Dimethylaminopropyl)trimethoxysilane, or [3-(2-aminoethylamino)propyl]trimethoxysilane) and hydroxyalkyl-, amino-alkyl-, or carboxyalkyl-functionalized thiols (for metal surfaces, such as Au, Ag, Cu, Ni, Zn, or Pt, and the like; (e.g. 11-Mercaptoundecyl)tetra(ethylene glycol); 11-mercaptoundecyl)-N,N,N-trimethylammonium bromide; 11-Mercapto-1-undecanol; 11-mercaptoundecanoic acid; 11-mercaptoundecylphosphoric acid). As used herein, "hydrophilic" means that the location has a water contact angle less than 40 degrees as measured by the sessile drop method known to those skilled in the art. Such hydrophilic surfaces can promote wetting and spreading of reagents.

Such functionalizations may include use of a photoprotected hydroxyalkyl, aminoalkyl, and/or carboxyalkyl-functionalized siloxane which may be photopatterned according to methods known to those skilled in the art to locally generate hydrophilic surfaces at discrete locations on the substrate surface. Such functionalizations may also include use of a reactive surface functionalization, such as an epoxy or isocyanato group which may be reacted with a second molecule (e.g., 1, 2-diaminoethane) to generate a locally hydrophilic surface (e.g., (triethoxysilyl)propyl isocyanate or 3-glycidoxypropyldimethylethoxysilane).

'In certain embodiments, the surface of the silicon-containing substrate is functionalized to be hydrophobic. Examples of hydrophobic functionalizations include, but are not limited to alkyl-functionalized and fluoroalkyl-functionalized siloxanes (for silica surfaces; e.g., 1H,1H,2H,2H-perfluorooctyltriethoxysilane and dodecyltriethoxysilane) and alkyl-functionalized thiols (for metal surfaces, such as Au, Ag, Pt, and the like (e.g., 1-Dodecanethiol). As used herein, "hydrophobic" means that the location has a water contact angle greater than 90 degrees as measured by the sessile drop method known to those skilled in the art.

Such functionalizations to capture and isolate the expression product can be located on a second substrate, or can be located within the same discrete location on the first substrate. For example, an individual well (e.g., rectangular sized or large elliptical/circular) can be treated such that one portion of the well has a nucleic acid (e.g., DNA) printed, and another portion has a coating of an antibody to the expression product. An expressed protein can bind to this antibody to isolate the pure protein. That is, a "capture substrate" can be a separate substrate from the silicon-containing substrate, or may be a portion of the silicon containing substrate available for use as to capture expression products. In one non-limiting embodiment the "capture substrate" comprises one or more discrete locations on the substrate adjacent to the discrete location at which expression occurs, and the locations are physically confined by a well or a tube (bottom capture, tube-wall capture or side capture etc.).

In another aspect, the disclosure provides methods for in situ nucleic acid expression, comprising
(a) contacting each discrete location of the array as described in any of the preceding aspects and embodiments thereof, with reagents for nucleic acid expression to form a mixture; and
(b) incubating the mixture under conditions suitable for nucleic acid expression.

In yet another aspect, the disclosure provides methods for expressing and capturing an expression product, comprising
(a) contacting a nucleic acid array with reagents for nucleic acid expression to form a mixture, wherein the nucleic acid array comprises a first substrate and at least 10 isolated coding nucleic acids physically confined at a discrete location on a first substrate, and wherein each nucleic acid is capable of expressing its encoded product in situ at its discrete location on the first substrate;
(b) optionally, the first substrate may be brought into the proximity of a capture substrate;
(c) incubating the mixture under conditions suitable for production of nucleic acid expression products; and
(d) capturing and isolating the expression products on the first substrate or, when present, the capture substrate.

It is well within the level of those of skill in the art to determine the most appropriate conditions for the contacting, incubating, and capturing steps based on the teachings herein. In one non-limiting example, conditions for NAPPA typically comprise incubating the DNA of 1-2 hours at 30°C to express proteins.

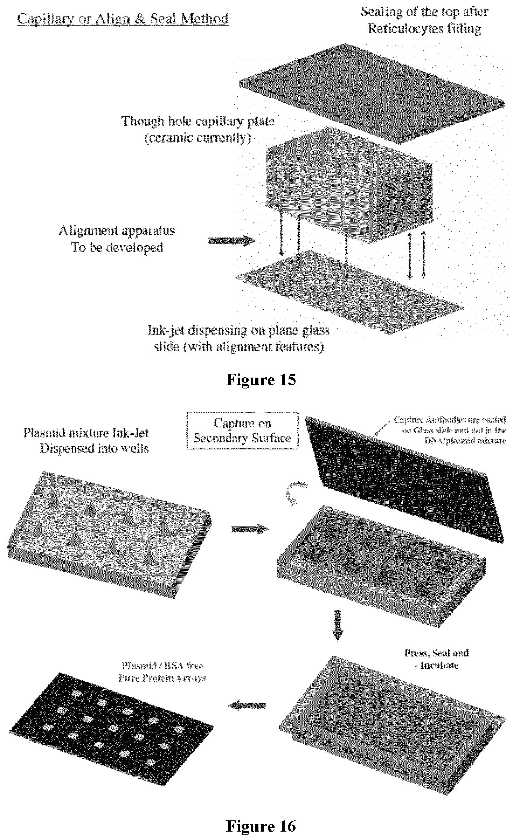

The expressed product may be captured on the same first substrate or the capture substrate. For example, each of the discrete locations may further comprise a biomolecule capable of binding to or associating with the expressed product, as discussed herein, such that the expression product is produced and captured at the discrete location. In another example, the first substrate may be contacted with a capture substrate, where the capture substrate is coated with a biomolecule capable of bonding to or associating with the expressed product; upon expression, the product can be captured on the capture substrate. Exemplary capture substrates and their use are provided in Figures **16-17****.** **Figure 16** (well) and **Figure 17** (capillary tubes) show embodiments in which capture antibodies are coated on glass slides (and not in the DNA/reagent mixture in the wells) that act as the capture substrate; contacting this capture substrate with the array and carrying out protein expression results in a capture substrate that is a pure protein array and does not contain the stating DNA or reagents. The capture substrates may be made of any material or comprise any other components suitable for a given use, including but not limited to gold and/or silver coated capture substrates (for example, for use in surface plasmon resonance (SPR) studies), capture substrates that comprise field effect nanowires (for example, to produce substrates having nanowires coated with the expressed proteins), and capture substrates comprising spin-coated probe molecules magnetic micro/nanoparticles coated with secondary antibodies or chemical linkers.

Using the methods of the invention, expression products can be expressed multiple times from the same array, enabling more than one isolated-expression product capture per array. Herein, 'isolated' means that the DNA print location is separated from the capture location of the expression product and not contaminated by other biological material. (e.g., in regular NAPPA both these locations are the same).

The contacting can be done under any conditions suitable for a given purpose. In one embodiment, the reagents can be flooded across all discrete locations. In another embodiment, the reagents can be discretely delivered to some or all discrete locations, for example, using known microfluidic techniques. As will be understood by those of skill in the art, the arrays may be generated and used immediately, or the arrays may be generated and stored for later use. When arrays are generated and stored for later use, the reagents (such as reticulocyte lysate) may be added at the time of use or the array/reagents may be generated and stored frozen, such that at the time of use the lysate is unfrozen and incubated as appropriate. In a further embodiment, the arrays may be frozen after the incubating/protein expression and any desired subsequent steps, for later use of the expressed proteins.

In various non-limiting embodiments of any of the preceding aspects and embodiments thereof, the methods may comprise one or more of the following:
▪ (a) Filling and sealing the discrete locations (such as nanowells) during polypeptide expression to prevent cross contamination between neighboring wells. For example, plasmid DNA in the nanowells is exposed to reagents, such as reticulocyte lysate, to express proteins from the DNA (e.g., use pre-vacuumed PDMS wells (or another foamy substrate like silicone rubber foam), where residual vacuum sucks lysate in; or e.g., fill wells with water first, remove water other than inside of wells, and then inject lysate to displace/mix water with lysate).

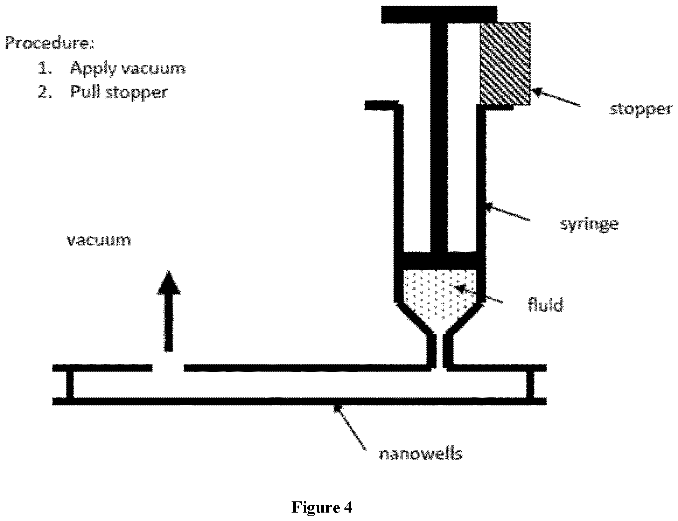

∘ (b) using a rigid cover with spacers between a cover (as used herein, including but not limited to a capture substrate) and substrate array surface, followed by removal of the spacers to push down the cover-plate and seal the discrete locations;
∘ (c) using a rigid cover-plate with elastic spacers between a cover and the discrete locations, followed by pushing down on the cover-plate to compress the elastic spacers to seal the discrete locations;
∘ (d) using a flexible cover and pushing down on a cover to deform it and seal the discrete locations;
∘ (e) forcing the reagents, such as reticulocyte lysate fluid through a narrow gap between the substrate and a cover to spread the fluid uniformly over the array surface;
∘ (f) filling the discrete locations (such as nanowells or tubes) with reagents, such as reticulocyte lysate using vacuum, syringe and solenoid-valve. See, for example, **Figure 4****.** Assembling a cover-plate, with two holes, over the substrate. Sealing the cover-plate to the substrate around the edges. Leaving a gap between the cover-plate and the substrate. Attaching a syringe to a solenoid-valve with just enough reticulocyte lysate to fill the discrete locations, the gap over the substrate and the assorted valves, fittings and tubing. Attaching the other end of the solenoid-valve to one of the holes in the cover-plate. Appling a vacuum to the other hole in the cover-plate. Opening the solenoid valve. Releasing the vacuum. This embodiment can be varied by one or more of:
   ▪ (1) replacing the solenoid-valve with a septum and piercing the septum with a syringe needle attached to the syringe to start the flow of reagents into the device;
   ▪ (2) eliminating the solenoid-valve, holding back the flow of reagents using a mechanical stopper on the syringe plunger and then releasing the mechanical stopper to start the flow of reagents into the device;
   ▪ (3) eliminating the solenoid-valve, holding back the flow of reagents using a syringe pump and then moving the syringe pump forward to start the flow of reagents into the device;
   ▪ (4) placing a flow restrictor in line with the solenoid-valve to slow down the flow rate if necessary;
   ▪ (5) adding mechanical damping to slow down the movement of the syringe plunger if necessary;
   ▪ (6) adjusting the gap between the substrate and the cover-plate to obtain uniform filling of all of the discrete locations; and
   ▪ (7) attaching a single syringe of reticulocyte lysate to multiple arrays to simultaneously fill more than one array at a time.
∘ (g) orienting the substrate vertically and submerging it, at a controlled rate, into a reagent thereby displacing air from the nanowells by the reagent;
∘ (h) orienting the substrate vertically and submerging it, at a controlled rate, into a reagent while vibrating the reagent to further assist in displacing air from the nanowells by the reagent;
∘ (i) placing the substrate vertically into a vessel and filling the vessel from the bottom at a controlled rate with a reagent thereby displacing air from the nanowells by the reagent;
∘ (j) placing the substrate vertically into a vessel and filling the vessel from the bottom at a controlled rate with a reagent while vibrating the reagent thereby further displacing air from the nanowells by the reagent;
∘ (k) filling the discrete locations with reagents, such as reticulocyte lysate inside of a vacuum chamber. Assembling a cover-plate, with one hole, over the nanowell array surface. Sealing the cover-plate to the nanowell array surface around the edges. Leaving a gap between the cover-plate and the nanowell array surface. Placing a drop of reticulocyte lysate over the hole and putting the assembly into a vacuum chamber. Appling a vacuum to the vacuum chamber and waiting until all of the air from the gap escapes through the small hole in the cover plate. Removing the vacuum thus forcing the reticulocyte lysate into the small hole and onto the nanowell array surface.
∘ (l) Filling the discrete locations of the substrate with reagents while the substrate is vibrating to release any air bubbles entrapped within the discrete locations.
∘ (m) Adding a lip around each discrete location (such as nanowells) with a lip for improved sealing.
∘ (n) Adding a thin film (which can optionally be transparent) between a silicone rubber and the wells.
∘ (o) Using a thin film and air pressure to seal the wells instead of silicone rubber.
   ∘ Regulate the temperature of the liquid for thermal cycling of the reaction process.

In another embodiment, a microsieve membrane can be used to cover the array. For example (relating to improved NAPPA), print NAPPA mixtures on the substrate. Using precise tooling, align the pores of the membrane to the NAPPA spots. Expose the NAPPA spots to reticulocyte lysate through the pores in the membrane to isolate protein expression within individual spots. Maintain an air-gap between the bottom of the microsieve-membrane and the top of the flat microarray surface. However allow the reticulocyte to bridge the gap. Make physical contact between the bottom of the microsieve-membrane and the top of the microarray surface. Use a highly perforated microsieve-membrane with very small, closely-packed pores so that multiple pores cover one NAPPA spot. This alleviates the need for precise alignment of the microsieve-membrane and the microarray surface. Based on the teachings herein, it is within the level of skill in the art to determine appropriate reagents and incubation conditions to use for an intended use.

"Capture substrate" as used herein can be a separate substrate from the first substrate, or may be a portion of the first substrate available for use as to capture expression products. In one non-limiting embodiment the "capture substrate" comprises one or more discrete locations on the substrate side by side with the discrete location at which expression occurs, and the locations are physically confined by a well or a tube (bottom capture, tube-wall capture or side capture etc.).

In one embodiment, the capture substrate comprises a second substrate that comprises a plurality of physically confined discrete locations that match the physically confined discrete locations on the first substrate. The capture substrate can be one or more further substrates, as appropriate for any intended use. Any arrangement of discrete locations on the second substrate can be used that is suitable for a given expression product capture method. In one non-limiting embodiment, each discrete location on second substrate comprises pre-patterned locations/array of devices or chemical patterns or material film patterns. In various non-limiting embodiments, gold nanodots can be used to generate plasmonic sensors, nanowire arrays can be used to generate field effect sensors, and meso-porous alumina/gold pads can be used to generate electrochemical sensors.

In another non-limiting embodiment, each discrete location on the second substrate comprises an array of nano-holes with lipid bi-layers, wherein lipid bi-layers capture expressed polypeptides to produce membrane polypeptides.

In a further non-limiting embodiment, multiplicities of second discrete locations are provided on different capture substrates. In one non-limiting example of this embodiment, multiple capture substrates can be used for each nucleic acid array (e.g., reusable)

In yet another non-limiting embodiment, the discrete locations comprise surfaces of micro or nano particles introduced into the confined spaces on the capture substrate. In one example of this embodiment, gold or magnetic micro/nano particles can be introduced into the discrete locations to create particles where surfaces are covered with individual polypeptides after capture.

In various further embodiments, the capture substrate is applied/subject to any one, or a combination of, a varying potential/voltage/current or application of a magnetic field or application of a temperature or a mechanical force, as appropriate for an intended use. In one example, electrochemical oxidation/reduction can be used to induce cover capture on metal or dielectric substrates.

In another embodiment, the cover-capture surface can be nano-structured to increase surface capture area and/or surface roughness, there-by binding more expression product, for increased signal. In another embodiment, etched/frosted glass (chemical or physical etch) can be used to provide a rough surface for coating with capture reagent.

In certain embodiments of any of the preceding methods, the first substrate or the capture substrate can be precoated with a first member of a binding pair, wherein the expression products comprise a second member of the binding pair, and wherein the incubating is done under conditions suitable for binding of the first member and the second member of the binding pair, resulting in capture of the expression product on the precoated substrate.

Any suitable binding pairs can be used for a given purpose. In one example, the substrate is pre-coated with biotin, and the biomolecule expression product comprises a streptavidin tag. Once the sequence for the streptavidin tag is expressed, it binds the expressed protein to the biotin-coated substrate. As will be apparent to those of skill in the art, many such permutations are possible, all of which are contemplated herein. For example another suitable binding pair comprises halotag protein and halotag ligands. In another example an E-coil tag on the expression product binds to a K-coil peptide immobilized on the substrate.

In other embodiments of any of the preceding methods, the first substrate is precoated with a first member of a binding pair. In other embodiments of any of the preceding methods, the capture substrate is precoated with a first member of a binding pair.

The capture substrate can be contacted to the first substrate to form a seal. In this embodiment, a seal is made so that each of the discrete locations is completely separated from each other. In certain embodiments, each discrete location comprises a well. In certain other embodiments, each discrete location comprises a tube. The tubes may be of any suitable type for a given purpose. In one embodiment, tubes can be commercially obtained from, for example, Incom or other vendors, as noted above.

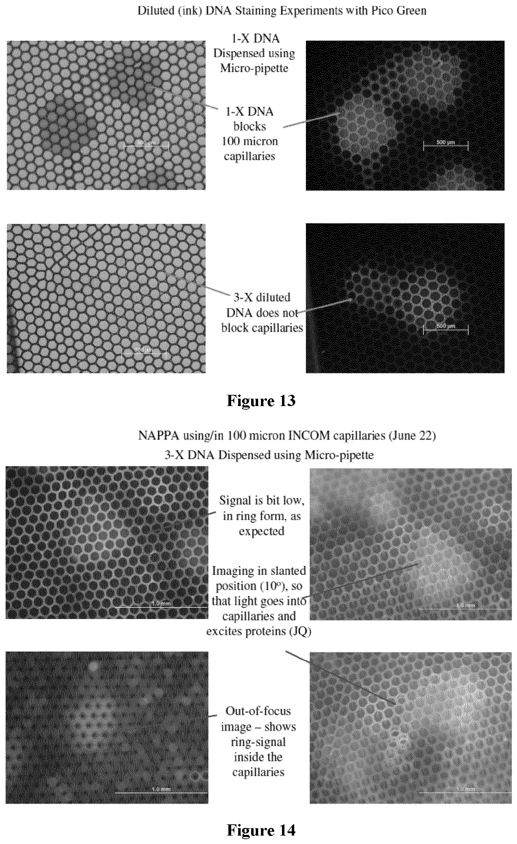

In one embodiment, NAPPA mixtures are dispensed into the tubes in an array format. For example, the inside of the tubes can be flooded with reticulocyte lysate to express polypeptides and capture them on the inside walls of the tubes. Optionally, both ends of the tubes can be sealed during polypeptide expression and capture. For detection, the array can be imaged by any suitable technique, including but not limited to illuminating the array from one-end and detecting fluorescent signal at the other end taking advantage of the light-guide properties of the arrangement. In another embodiment, a user can optionally hold the light-source at an angle to increase signal strength **(****Figure 12****).** Exemplary methods for expressing a product within a tube array **(****Figure 13****)** and for detecting the presence of fluorescent molecules within and on the surface of the tubes (Figure 14) are provided. cDNA was diluted with reagent mix and placed into the tubes. The tubes were then filled with reticulocyte lysate and expressed as usual in NAPPA. When the NAPPA print mix is dilute enough the print mixture with cDNA is deposited on the side of tubes. If the print mix is too concentrated, it may block the tubes, making it more difficult to introduce the lysate. cDNA coating on the side of the tubes facilitates filling with lysate and expression. In this case the final image shows a clear internal ring of fluorescent signal, inside of the tubes corresponding to printed locations.

In certain embodiments, nucleic acid expression comprises RNA expression. In other embodiments, the nucleic acid expression comprises polypeptide expression. Accordingly, the reagents may comprise an in vitro expression system. Any suitable in-vitro expression system can be used, including: but not limited to reticulocyte lysates, insect cell lysates and human cell lysates.

The method may further comprise modifying the polypeptide capture on the capture substrate. Any suitable modification of the polypeptide can be made as appropriate for an intended use. In one non-limiting embodiment, the bound/captured polypeptide is post-translationally modified using the same or a secondary reagent that may be present in the expression mixture or added after the expression-capture. This embodiment may be carried out on the capture substrate only, or may include a third substrate. The method may further comprise stabilizing the captured polypeptides on the capture substrate to limit protein diffusion. Stabilizing may be, for example but not limited to, freezing the captured polypeptides on the capture substrate.

The captured proteins on the capture-reagent coated surface may be used to carry out one or more process selected from the group consisting of surface plasmon resonance (SPR) detection, MALDI mass spectrometry, and post-translational modification.

In another aspect, the invention provides methods for detecting biomolecules on any of the arrays described in the preceding aspect and embodiments thereof, comprising, scanning the arrays at various depths and combining the intensity reading at each pixel of the resulting scanned images. As used herein, 'combining' means calculating any suitable mathematical norm of the pixel intensities, including but not limited to the average or peak value, for the purpose of uniformly increasing the detection signal across discrete locations. Typical microarray scanners used for fluorescent detection of biomolecules are designed to scan a flat surface and consequently have limited depth of field. However the discrete locations disclosed here may be three dimensional. Scanning these locations at various focal depths and then combining the resulting images circumvents limitations due to the limited depth of field of microarray scanners for this application.

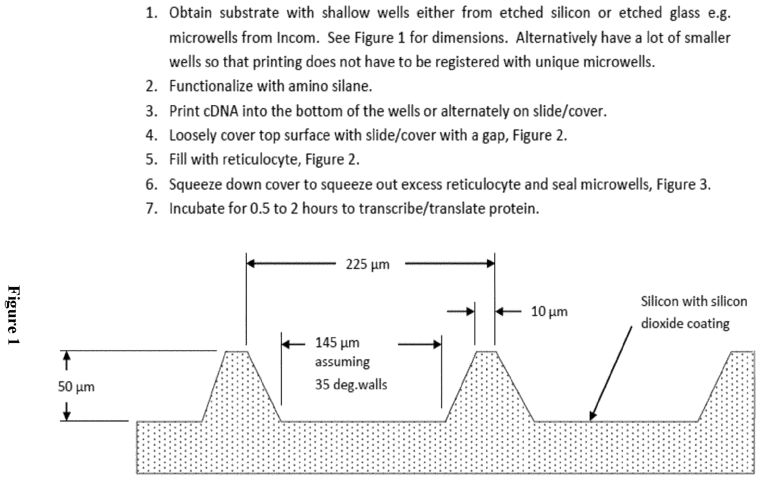

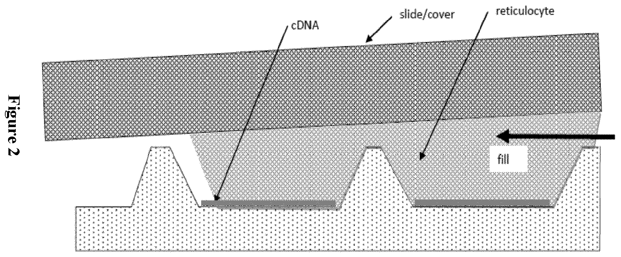

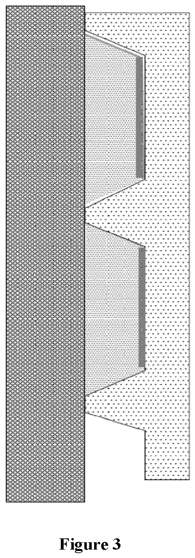

One non-limiting embodiment of the arrays of the invention and their preparation and use is provided in **Figure 1****,** and has 50 µm depth wells that have 35° walls and 145 µm diameter, where the wells have a 225 µm period. Substrates containing such arrays can be obtained, for example, from etched silicon or etched glass. The wells can be functionalized with amino silane prior to printing of cDNA on the bottom of the wells (or alternatively the cover) and filled with reticulocyte lysate, followed by use of a cover to squeeze out excess reticulocyte lysate and seal the microwells **(****Figures 2-3****).** The arrays can then be incubated for 0.5 to 2 hours to transcribe and translate the protein encoded by the cDNA.

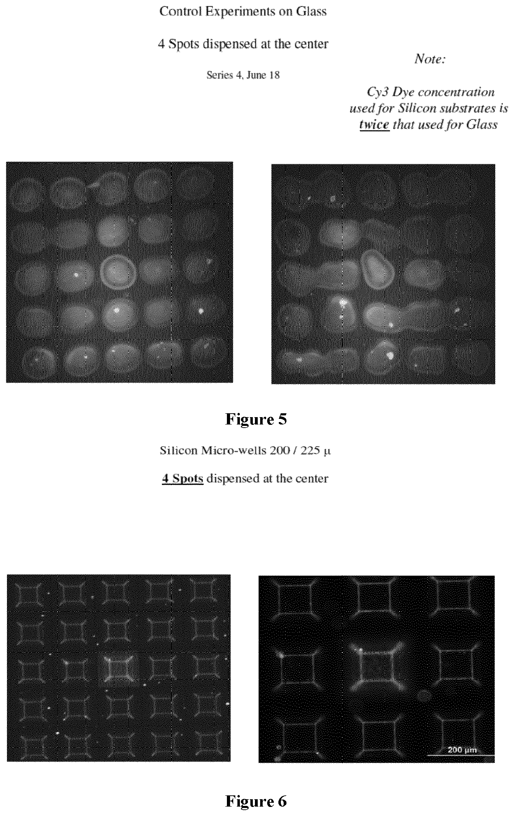

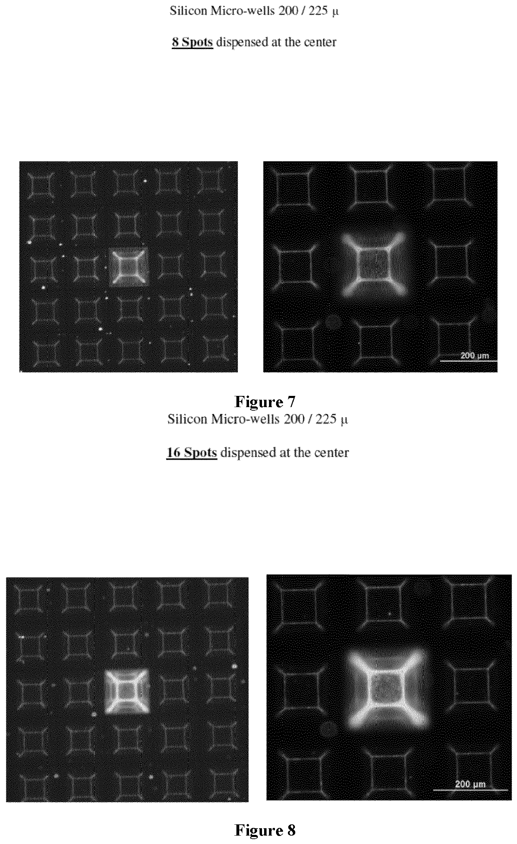

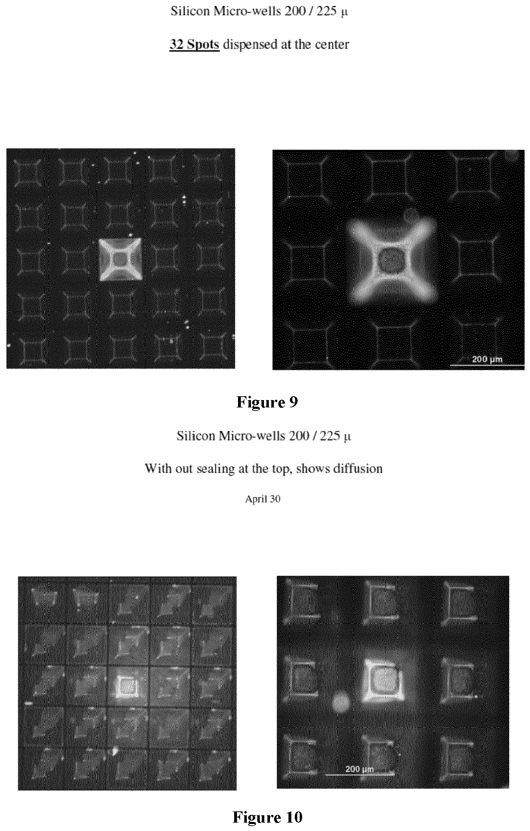

**Figures 5-10** show the results of experiments in which a controlled amount of DNA and reagents are dispensed either on glass or in wells, showing the significant improvement in reducing diffusion using the arrays and methods of the invention. **Figure 5** shows a control experiment on glass, with four drops of DNA & reagents dispensed in the center spot, proteins produced from the DNA using reticulocyte lysate and the proteins tagged with fluorescently labeled antibodies; significant diffusion between spots is evident in the detected fluorescence pattern. **Figure 6** shows the same experiment as **Figure 5** using sealed silicon nanowells with a period of between 200-225 µm instead of glass, with a resulting significant reduction in diffusion of fluorescence from one well to another. **Figures 7-9** show similar results using increasing amounts of DNA and reagents per well, while **Figure 10** shows that the cover seal helps to limit diffusion between wells.

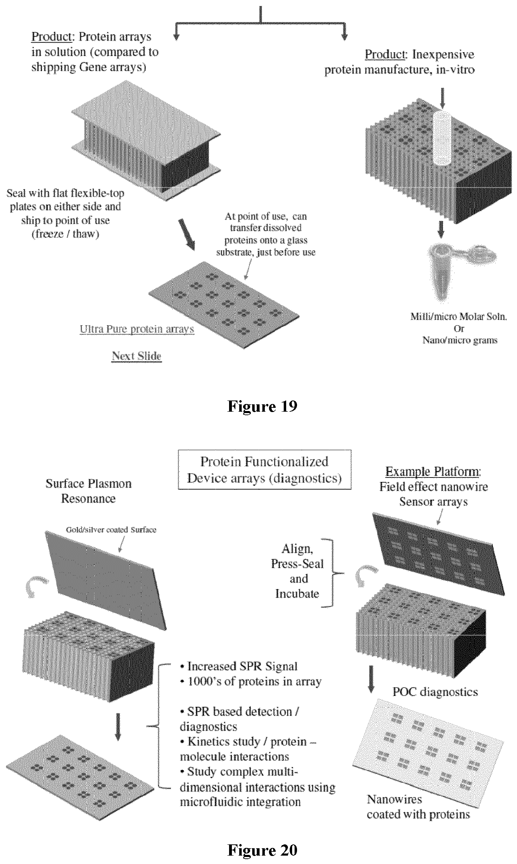

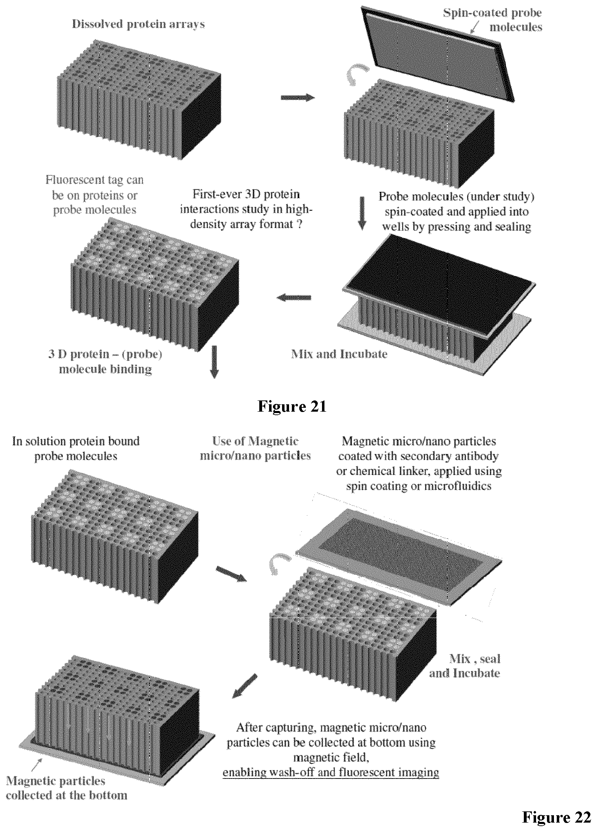

In one embodiment employing microcapillaries, **Figure 18** shows a method for releasing a captured expression product from a substrate comprising microcapillaries via cleavage of a cleavable linker (chemical or photo-induced), or by competing with excess antibody or antigen. The expressed proteins are thus suspended in solution, producing an array of proteins in solution phase, which can be stored in any suitable manner. For example, the array can be sealed with sealing/capture substrate on either or both side of the array and frozen for storage or shipment **(****Figure 19****).** When ready to be used, the user can transfer the proteins onto the substrate just before use, or can use the arrays in any other suitable manner. In other embodiments, the arrays can be sealed with a capture substrate (including but not limited to gold and/or silver coated surfaces) suitable for use in surface plasmon resonance (SPR), allowing the resulting arrays on the capture substrate to be used in SPR-based detection/diagnostics or other suitable studies. Alternatively, the array can be sealed with a capture substrate that comprises field effect nanowires, where the resulting capture substrate has nanowires coated with the expressed proteins, for use in diagnostic or other suitable sensing procedures **(****Figure 20****).** Other exemplified capture substrates include those with spin-coated probe molecules or capture substrates with magnetic micro/nanoparticles coated with secondary antibodies or chemical linkers and applied to the capture substrate using spin coating or microfluidics. **(****Figures 21-22****)**

Those of skill in the art will understand that all of these exemplified embodiments can also be used with well-based embodiments of the arrays, or any other embodiment of the means for physical confinement.

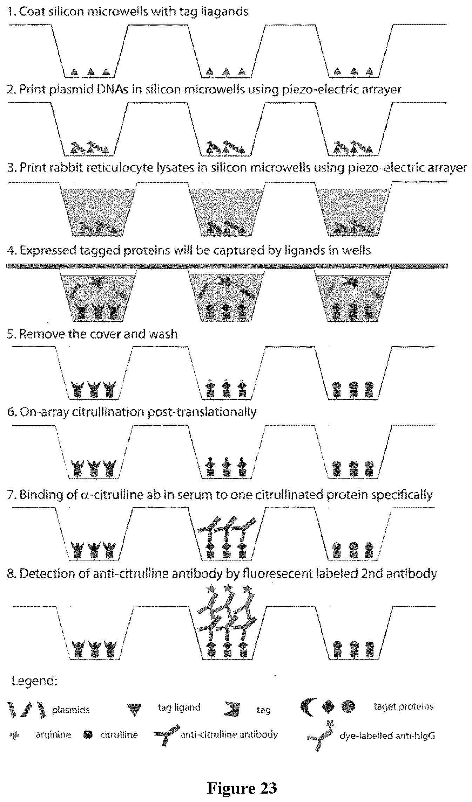

**Figure 23** shows an embodiment for capturing an expression product within the same nanowell where expression occurred. In this embodiment, silicon nanowells are coated with ligands for a tag expressed as part of the protein product to be expressed. Plasmid DNA encoding the tagged expression products of interest is printed in the microwells via any suitable technique, such as via a piezoelectric arrayer. Reticulocyte lysate is then placed in the wells via any suitable technique, such as by flooding all of the wells or dispensing into individual wells using a piezoelectric arrayer, and the wells are sealed. The array is incubated under suitable conditions for protein expression, and expressed tagged proteins will be captured by ligands in the wells. The sealing means is removed and the wells washed under conditions suitable to remove unbound materials. Bound proteins are contacted with any suitable reagent, such as reagents/conditions suitable for citrullination of the bound proteins, which can then be detected by incubation under suitable conditions to bind anti-citrulline antibodies/fluorescently labeled secondary antibodies.

### Examples

In this study, we sought to determine whether *in situ* synthesis of NAPPA reactions suffered from diffusion-related cross-talk at higher array densities. We then sought to solve the problem of diffusion with an innovative silicon nanowell platform that used the NAPPA protein arrays system as a test case. This platform enables confined biochemical reactions in physically separated nanowells. The NAPPA method was adapted to nanowell array substrates produced using silicon micro fabrication technology, which enables high-throughput, high-fidelity fabrication of nanowell substrates. We have also simultaneously developed a precise and accurate high-throughput liquid dispensing system to align and dispense genes and reagents into individual wells. After *in vitro* expression of proteins in the nanowells with a sealed cover, we demonstrated successful protein display in wells with negligible diffusion. Preliminary results also indicated functional protein that allows detection of known protein-protein interactions. Our development represents a major step forward in the production of functional human proteome protein arrays without diffusion of soluble species from feature to feature.

### Results

### NAPPA on glass slides

We tested the effect of reduced spacing of features using NAPPA. Diffusion of expressed proteins captured at neighboring locations became significant as separation distances (center-to center) drop below 400 microns on NAPPA. This is demonstrated in **Figure 24** where genes were printed on planar glass in two different array densities: a low density array with a 750 micron period **(****Figure 24b****)** and a high density array with a 375 micron period **(****Figure 24c****).** The features were printed in a pattern such that the center feature, containing cDNA + printing-mix, was surrounded by control features, where the control features contained only capture antibody (no genes). This configuration is highly sensitive for detection of cross-contamination because the control features do not produce protein that could compete with diffused protein. As illustrated in the 3-D rendering **(****Fig. 24****),** there was significant diffusion to neighboring features at 375 micron spacing compared to minimal diffusion for the 750 micron period array. The halo of signal around the center spot seen in both images is probably due to protein diffusion followed by physisorption to amine coated glass surface, and the shift of halo off-center may be due to fluid drift.

### NAPPA in silicon nanowells

To enable high density printing without diffusion, we replaced planar glass slides with slides comprising an array of nanowells on the silicon substrate and sealed the wells during protein expression **(****Figure 25****).** Adapting the NAPPA method to the nanowell platform enables physically confining protein expression and the ensuing antibody capture in nanoliter volumes (volume of nanowells ≤ 5 nanoliter). Expressed proteins are free to diffuse within the individual sealed wells until they are captured by the anti-GST antibodies in the wells. Semi-spherical nanowell arrays, approximately 250 microns in diameter and 75 microns deep with a period of 375 microns, were fabricated on silicon wafers, diced into the shape of glass slides (1 inch x 3 inch), and used as substrates for protein display. Monolithic crystalline silicon wafers were chosen due to the established silicon processing techniques that allow for well-controlled and inexpensive fabrication of nanowell array slides. Wells were etched by photo-patterning silicon-nitride mask layer deposited on silicon, using HNA isotropic etch chemistry (see also Materials and Methods). Nanowells etched in silicon were coated with 100 nm of dry oxide grown at 1,000 C in an oxidation furnace. Semiconducting silicon acts to quench surface fluorescence due to its semimetallic nature, hence requiring 100 nm thin layer of oxide dielectric layer. Additionally, thermally grown silicon dioxide serves as high quality glass surface for subsequent aminopropyltriethoxy silane (APTES) coating and appropriate NAPPA chemistry.

### Non-contact piezoelectric dispensing

Standard solid pin printing would not suffice for the precision that is required in printing expression mixtures into the nanowells. Thus, we developed a new method using piezoelectric printing. NAPPA expression mixtures were piezo-jet dispensed into APTES coated silicon nanowells (SiNW) using Engineering Arts' 8-tip au302 piezo printer, capable of aligning and printing at the center of the nanowells at very high-speeds **(****Figure 25c****).** One of the key challenges of nanowell technology is precise alignment and dispensing of many "unique" printing solutions onto a batch of nanowell slides in a suitable time frame. Special dispense hardware and software were developed that utilized the 8 head non-contact "on-the-fly" dispense technology resulting in a batch processing time of a few hours to fill ∼7,000 wells each slide, on a batch of up to 8 nanowell slides (details in methods section).

### Vacuum assisted SiNW filling of nanowells

After printing, NAPPA SiNW slides were first subjected to vacuum infiltration by de-ionized water for 5 to 10 minutes followed by vacuum infiltration by SuperBlock TBS (Thermo Scientific) solution for 5 to 10 minutes and subsequently incubated at room temperature and atmospheric pressure on a rocking shaker for 30 to 60 minutes. The slides were then rinsed thoroughly with de-ionized water and dried under a gentle stream of filtered compressed air. Any unbound or loosely bound material (DNA, anti-GST, BSA, BS3 crosslinker or trace DMSO) should wash away during these pre-hybridization blocking and washing steps; thereby minimizing the chance for material to break loose during subsequent steps. After blocking, the SiNW slides were incubated with rabbit reticulocyte lysate-(RRL) based *in vitro* transcription and translation (IVTT) system for protein expression and capture *in situ.* When this viscous lysate was directly introduced onto a slide with an array of nanowells, it exhibited a tendency to flow over the nanowells entrapping air without filling the wells (data not shown). This is an expected behavior due to liquid surface-tension where cohesive-forces tend to minimize the liquid surface area. To address this problem, we developed a vacuum-assisted filling procedure **(**Figure 25 d,e), which works independently of: the size and shape of nanowells; the fluid properties of filling liquid; or the properties of nanowell substrate.

In this procedure an enclosed air-tight micro-chamber is created over the nanowell slide by sandwiching the SiNW slide between a gasket cutout and two planar surfaces as shown in **Figure 25** **d.** An inflexible metal plate forms the bottom planar surface, while the top surface is made by sticking a thin film of flexible transparent silicone on thick inflexible glass slab held on a metal frame. Two 1.5 mm wide 100 micron thick adhesive-backed plastic strips (not shown in schematic) are applied manually along the long edges of the SiNW slide. The assembly is clamped on all sides and pressed together to reduce the height (thickness) of air-tight microchamber to approximately 100 microns, with the top surface compressing the surrounding gasket cutout and resting on the side strips on SiNW slide.

Lysate was introduced into the syringe attached to the port (a 1 mm diameter hole on top plate) as shown, and was held in-place inside the syringe due to the airtight micro-chamber (Figure 25 d). Air inside the micro-chamber and air dissolved in the lysate solution was removed by applying a gradual vacuum (up to 28 inches Hg) for 2 minutes. Using a three way solenoid valve (adapters and solenoid not shown) the syringe was then instantly switched from vacuum to atmospheric pressure. Once switched, the pressure difference, i.e., atmospheric pressure acting on the lysate solution against vacuum in the wells, drove the liquid into filling all the nanowells effectively in less than two seconds. This in-house developed vacuum-assisted filling system ensured a good pressure seal by virtue of the *transparent flexible silicone* film supported on glass slab (inset image of sealed silicon nanowells, Figure 25 e). The silicone film, under applied pressure, conforms around the narrow top edges of wells, to seal the nanowells. The sealed assembly was then incubated for protein expression and binding.

### Minimal protein diffusion between nanowells using SiNW

To test protein expression and capture in silicon nanowells and to assess the level of cross-talk between the nanowells, sixteen different genes were printed into every other nanowell, (schematic of print pattern in **Figure 26a****).** Intervening wells were left empty, and signals observed in these intervening wells would indicate the level of cross-contamination due to diffusion. Pico-green staining of printed DNA showed the expected alternate fluorescent signals **(****Figure 26b****),** confirming precise "on-the-fly" dispensing by the piezo printer into the wells. When the nanowells were left un-sealed (without clamping) during protein expression, spillover signal was observed in intervening wells **(****Figure 26c****).** Whereas, display of expressed and captured proteins in sealed nanowells is shown in **Figure 26d****.** As seen from the image and its 3D signal profile, there was no discernible diffusion of proteins from expression wells to neighboring empty wells.

### High Density NAPPA Protein Array

After successfully demonstrating precise dispensing and confined expression with a small number of genes, we produced SiNW slides with an array of 8,000 nanowells (SiNW-8K chip, 375 um feature distances). These were used to confirm diffusion-free expression across the footprint of a full size microscopic slide for a large number of genes in sealed nanowells (not shown). Two-hundred eighty-seven (287) randomly selected genes, 192 from *Vibrio cholerae* and 96 from human, were printed in blocks of 6 rows x 48 columns, which was repeat printed 24 times.. TP53, FOS and JUN proteins were interspersed in the block pattern, with p53 protein repeat printed twice in each block. The DNASU logo was printed by pooling 8 different genes into a single composition (no p53). The print pattern also included empty nanowells around the above array, and surrounding the logo at the bottom, as negative controls. Consistent and precise dispensing across the whole array was shown by the picogreen staining of printed DNA (not shown). Overall, the array showed consistently high protein expression as detected by anti-GST staining.

### Diffusion-free high density protein arrays

The principle aim of this work - to solve the issue of diffusion in very high density arrays - has been successfully addressed. Almost all empty spots around the print block and empties surrounding the DNASU logo showed no significant signal above background.

To further confirm diffusion-free protein display, we used a more sensitive test to assess diffusion by probing the high density NAPPA in SiNW with antigen specific antibodies against TP53 protein to determine if its signal was observed in neighboring wells (not shown). The ratio of average signal from neighbor spots to average signal from cognate p53 spots was calculated to be just 1.34%. This confirms that very high density in-situ protein arrays can be successfully produced using nanowells to arrest protein diffusion and cross-binding. Analyzing the p53 signals, the coefficient of variation (CV) between the 48 repeat spots is calculated to be 12.84%. Discounting the one outlier low-signal p53 spot on bottom right side, the CV was calculated to be 8.5%.

### Functional studies on nanowell protein arrays

To investigate the functionality of the proteins produced in nanowells, we examined protein-protein interactions using the well-established FOS-JLTN interaction (16) as a surrogate assay for proper folding and functionality of proteins expressed on SiNW slides with the same printing pattern as above (not shown). Query DNA that encoded HA-tagged FOS was mixed in the IVTT lysate mixture and co-expressed with the array proteins. Antibodies to FOS or HA-tag were then used to reveal specific interactions of FOS with JUN displayed on the array. The query protein HA-FOS did not have the GST tag and could not be captured by anti-GST antibody co-spotted in each well. HA-FOS would be detected only if they could bind to the captured array target proteins tethered to the well.

Highly specific antibodies were selected for the interaction study. If no query DNAs were added to the expression system, anti-HA did not detect any reactivity and anti- Fos only detected FOS spots on expressed arrays (not shown). The studies also further confirmed confined expression of FOS in individual wells on silicon nanowell arrays using above described vacuum assisted filling and sealing method, and showed specific Fos-Jun interaction with HA-FOS protein as a query and detected by an anti-Fos antibody. As antibodies against proteins of interest are not always available, we also demonstrated detection of protein interactions by using antibodies against the HA tag on the query protein (not shown).

### Ultra-high density protein arrays

To determine if the silicon nanowell platform is also compatible with a human proteome-on-chip scale, we produced two versions of ultra-high density arrays with 24,000 features. Displaying 24,000 proteins on a single array requires nanowells with array spacing of 225 microns or below. 225 micron period silicon nanowells were produced in both round-well and square-well geometries **(****Figure 27****).** While the round nanowells were produced using HNA etch chemistry, square nanowells were produced using KOH anisotropic etch chemistry on Si (100) wafers with well-depth of approximately 100 microns. Etch anisotropy of square wells which produces deep wells with no significant lateral etching is of interest for further higher density protein arrays. Protein expression and capture were tested in these chips using the standard protocol. Comparable signals were achieved on these 24,000 feature arrays relative to those on the 8,000 feature arrays indicating robust protein expression on these ultra-high density arrays and relieving concerns of potential expression lysate exhaustion in smaller volume wells. Bright signals along the edges of the square nanowells in **Figure 27f** are speculated to be due to preferential aggregation of proteins and dye at the sharp edges. Sequential KOH anisotropic etching followed by short-duration HNA isotropic etching to round-off all the sharp edges is expected to solve this issue. It is notable that even at this density, there was no significant cross-talk signal in neighboring wells in both round and square nanowell geometries, confirming effective limiting of diffusion in sealed silicon nanowells compatible with proteome-on-chip technology.

### Discussion

Establishing a platform to study protein biochemical properties in a multiplexed and high-throughput fashion is important for many different biomedical research areas. Protein microarrays represent one such platform. NAPPA is an innovative alternative to conventional protein arrays and bypasses the challenges associated with protein expression, purification and storage. NAPPA is a particularly flexible protein microarray format because a customized array can be created simply by re-arraying a series of plasmids encoding proteins of interest. However, transcription and translation on a planar surface entails the presence of intermediates that can diffuse before capture by co-spotted capture reagent. Furthermore, planar surfaces are limited to assays that do not have diffusible products or reactants that need to remain local.

In this study, we have developed a silicon nanowell based protein microarray platform that not only addresses the cross-talk problem in high density NAPPA but also has the potential of performing other biochemical reactions in these nano-reaction vessels that are not possible on traditional planar protein array format. For example, the kinetics of enzymatic assays that release fluorescent products might be monitored directly in each well or various post-translational modifications could be performed on displayed proteins. Our NAPPA SiNW platform builds upon the mature semi-conductor industry for substrate micro fabrication. Although most of our experiments used a density of 8,000 features per standard glass slide with center-to-center distance of 375um, we do not foresee any obstacles of increasing densities many times higher and we have run proof-of-concept expression on arrays with 24,000 features.

We have observed minor regional variations in signal intensities within the curved nanowells. These variations are also observed on flat surfaces and probably represent non-uniform settling of precipitates during drying of microarray spots. Other contributors may include incomplete filling of the nanowells during printing causing some signal attenuation towards the edges, and/or non-uniform surface irregularities due to wet-etching of the nanowells.

### MATERIALS AND METHODS

### Micro fabrication of silicon nanowells

Six inch diameter Silicon <100> wafers were used as starting material for producing silicon nanowell (SiNW) slides. Each 6-inch wafer yielded 6 slides per wafer after dicing. In future, larger diameter wafers are expected to yield higher number of slides per wafer, making SiNW slides very inexpensive. Standard semiconductor processing techniques were used to fabricate the SiNW slides, as depicted in the schematic in **Figure 25****.** Silicon nanowell slides were fabricated at Arizona State University Center for Solid State Electronics Research. The wafers were first coated with 300 nm of LPCVD low stress nitride at 835 C, which acts as a mask layer for wet etching of nanowells. Wafers were then spin-coated with 1 micron thick AZ 3312 positive photo resist (AZ Electronic Materials Inc), followed by soft bake on a hotplate at 100 C for 2 minutes. Photo lithography masks with circular features were used to expose the resist on an OAI photo mask aligner, for producing round nanowells of desired diameter and spacing. The photo resist was then developed in AZ300 MIF developer for 45 seconds, and hard-baked on hotplate at 100°C for 2 minutes. Reactive ion etching using CHF₃ - O₂ plasma was used to etch away the nitride film, and open circular array pattern on the nitride layer. Photo resist layer was washed away using acetone.

### Isotropic and anisotropic etching of nanowells

Isotropic etching of wells was selected as preferred method compared to anisotropic etching. While anisotropic etching has the advantage of producing wells of high aspect ratio, it results in sharp facets and edges inside the nanowell. It was observed that piezo-dispensed DNA/plasmid mixture and the expressed proteins both tend to aggregate and bind preferentially at these sharp edges. To attain a relatively uniform protein binding inside the wells a semi-spherical curved surface was produced using isotropic etching. Furthermore, etch mask and etch time were designed so as to produce a circular flat surface at the bottom of the semi-sphere, which acts as a substrate for dispensed DNA/plasmid mixture. Flat circular surface at the bottom of the semispherical wells has the advantage of distributing the dispensed DNA and the resulting protein binding, uniformly over the flat area, compared to a fully-spherical curved bottom which tends to aggregate these into a spot at the center.

HNA silicon etchant was prepared by mixing hydrofluoric acid (49%), nitric acid (70%) and glacial acetic acid (98%) in the ratio of 2.75:1.75:1. All chemicals were procured from Sigma Aldrich. HNA etchant is an extremely aggressive and corrosive mixture. It has to be prepared in special acid baths, and requires very careful handling with special disposal methods, by well trained personnel. HNA mixture etches silicon at an approximate rate of 3 microns per minute. 30 minute etch of silicon produced wells with depths ranging from 60 microns to 80 microns, depending on diameter of the circular openings in the nitride-mask. Anisotropic pyramidal wells **(****Figure 27f****,** proof of concept 225 micron period array) were produced by patterning square openings in nitride mask layer on Si (1000) wafers, and etching in 30% KOH solution at 80°C. Hot KOH is an aggressive chemical, strong corrosive, and needs to be handled by well-trained users. The sharp edges produced by anisotropic etching can be smoothed using a two-step process, with an HNA isotropic etch step following an anisotropic etch process.

Silicon surface at the bottom of the etched wells quenches fluorescent signal during assay, query detection in later steps. Hence a thin film of silicon dioxide was thermally grown that acts as a suitable dielectric and also mimics the glass surface of regular NAPPA arrays. For this purpose, wafers with etched wells were cleaned in Piranha mixture (1:1 mix of sulfuric acid and hydrogen peroxide) followed by a ten second clean in buffered oxide etch (1: 6 mixture of HF and NH₄F). Piranha mixture and buffered oxide etch are both very aggressive chemicals, to be prepared in special containers, and needs to be handled by well-trained users. A dry oxide of thickness 100 nm was thermally grown at 1,000°C in Tystar 4600 oxygen furnace. After oxide growth the mask nitride film is etched away in hot phosphoric acid (185°C). The resultant wafers have round wells coated with uniform 100 nm oxide thin film, with spacing in-between wells comprising of semi-metallic silicon surface. This structure has the additional advantage that fluorescent signal is emitted from glass-like the oxide coated wells, while the metal-like silicon surface in un-etched areas in-between the wells quenches any fluorescent emission (apparent in **Figure 26d** - no seal case), providing a good contrast in fluorescent imaging. Finally the wafers were diced into microscope-slide sizes yielding silicon nanowell (SiNW) substrates for high density NAPPA protein arrays.

### Amine functionalization of silicon dioxide surface

Prior to piezo-dispensing of DNA/plasmid mixture into the nanowells the surface of the wells was coated with amino-propyl-triethoxy-silane (APTES) monolayer. It has been demonstrated that for producing NAPPA protein arrays, an amine terminated surface that acts as suitable substrate to adhere to dispensed DNA/plasmid mixture is required. For this purpose SiNW substrates were first cleaned in Piranha mixture (1:1 H₂SO₄ and H₂O₂) for a period of 15 minutes. Piranha mix is a strong oxidant that cleans any residual organic materials on the SiNW substrates and oxidizes surface of silicon oxide to produce silanol (-SiOH) surface terminations. SiNW slides are then immersed in 2% solution of APTES in acetone, for a period of 15 minutes, followed by thorough rinse in acetone and DI water, to produce uniform monolayer of APTES molecules.

### High speed piezo printing in nanowells

Piezo printing was accomplished using an au302 piezo dispense system (see web site engineeringarts.com) with a newly developed integrated alignment system for nanowell slides. The alignment system consists of a micrometer angular alignment fixture, look-down camera, transfer arm and vacuum tray. Nanowell slides are aligned one at a time on the alignment fixture using the look down camera and then transferred with the transfer arm to the vacuum tray. A row of aligned nanowell slides placed on the vacuum tray can then be dispensed "on-the-fly" with the head moving at 175mm / sec resulting in a peak speed of 50 wells per second using 8 dispense head. Each nanowell is filled with 800 picoliters of printing solution (cDNA + printing-mix). Following piezo printing of cDNA in nanowell array, NAPPA SiNW slides were stored in dry, sealed container until the time of use.

### DNA preparation

Sequence-verified, full-length cDNA expression plasmids in the T7-based mammalian expression vector pANT7_cGST or pANT7-nHA were obtained from Arizona State University, Biodesign Institute, Center Personal Diagnostics, DNASU and are publicly available (see web site dnasu.asu.edu/DNASU/). The high-throughput preparation of high-quality supercoiled DNA for cell-free protein expression was performed as described (30). For protein interaction assay, larger quantities of query DNA were prepared using standard Nucleobond preparation methods (Macherey-Nagel Inc., Bethlehem, PA).

### Protein expression

Protein display was performed as described (9). Displayed proteins were detected using Tyramide signal amplification (TSA, Life technologies, Carlsbad, CA) with a monoclonal anti-GST antibody (Cell signaling Inc., Danvers, MA) and HRP-labeled anti-mouse antibody (Jackson ImmunoResearch, West Grove, PA). Anti-p53 monoclonal antibody (Santa Cruz Biotechnology Santa Cruz, CA) was used for p53 specific signal detection to assess diffusion.

### Protein interaction

Protein interaction was performed as described (9). FOS gene in pANT7-nHA was added to the RRL expression mixture at a concentration of 1 ng / ml. HA-tagged FOS bound to interaction partners on array was detected either by gene specific anti-FOS (Santa Cruz Biotechnology, Santa Cruz, CA) antibody or tag specific antibody (anti-HA, Convance) followed by Alexa fluor labeled secondary antibodies (Life technologies, Carlsbad, CA).

### REFERENCES

1. Gibson, D. S.; Qiu, J.; Mendoza, E. A.; Barker, K.; Rooney, M. E.; Labaer, J., Circulating and synovial antibody profiling of juvenile arthritis patients by nucleic acid programmable protein arrays. Arthritis Res Ther 14, (2), R77.
2. Anderson, K. S.; Sibani, S.; Wallstrom, G.; Qiu, J.; Mendoza, E. A.; Raphael, J.; Hainsworth, E.; Montor, W. R.; Wong, J.; Park, J. G.; Lokko, N.; Logvinenko, T.; Ramachandran, N.; Godwin, A. K.; Marks, J.; Engstrom, P.; Labaer, J., Protein microarray signature of autoantibody biomarkers for the early detection of breast cancer. J Proteome Res 10, (1), 85-96.
3. Lin, Y. Y.; Lu, J. Y.; Zhang, J.; Walter, W.; Dang, W.; Wan, J.; Tao, S. C.; Qian, J.; Zhao, Y.; Boeke, J. D.; Berger, S. L.; Zhu, H., Protein acetylation microarray reveals that NuA4 controls key metabolic target regulating gluconeogenesis. Cell 2009, 136, (6), 1073-84.
4. Loch, C. M.; Cuccherini, C. L.; Leach, C. A.; Strickler, J. E., Deubiquitylase, deSUMOylase, and deISGylase activity microarrays for assay of substrate preference and functional modifiers. Mol Cell Proteomics 10, (1), M110 002402.
5. Jones, R. B.; Gordus, A.; Krall, J. A.; MacBeath, G., A quantitative protein interaction network for the ErbB receptors using protein microarrays. Nature 2006, 439, (7073), 168-74.
6. Stiffler, M. A.; Chen, J. R.; Grantcharova, V. P.; Lei, Y.; Fuchs, D.; Allen, J. E.; Zaslavskaia, L. A.; MacBeath, G., PDZ domain binding selectivity is optimized across the mouse proteome. Science 2007, 317, (5836), 364-9.
7. Hudson, M. E.; Pozdnyakova, I.; Haines, K.; Mor, G.; Snyder, M., Identification of differentially expressed proteins in ovarian cancer using high-density protein microarrays. Proc Natl Acad Sci U S A 2007, 104, (44), 17494-9.
8. Ramachandran, N.; Hainsworth, E.; Bhullar, B.; Eisenstein, S.; Rosen, B.; Lau, A. Y.; Walter, J. C.; LaBaer, J., Self-assembling protein microarrays. Science 2004, 305, (5680), 86-90.
9. Ramachandran, N.; Raphael, J. V.; Hainsworth, E.; Demirkan, G.; Fuentes, M. G.; Rolfs, A.; Hu, Y. H.; LaBaer, J., Next-generation high-density self-assembling functional protein arrays. Nature Methods 2008, 5, (6), 535-538.
10. Wright, C.; Sibani, S.; Trudgian, D.; Fischer, R.; Kessler, B.; LaBaer, J.; Bowness, P., Detection of multiple autoantibodies in patients with ankylosing spondylitis using nucleic acid programmable protein arrays. Mol Cell Proteomics 11, (2), M9 00384.
11. Ceroni, A.; Sibani, S.; Baiker, A.; Pothineni, V. R.; Bailer, S. M.; LaBaer, J.; Haas, J.; Campbell, C. J., Systematic analysis of the IgG antibody immune response against varicella zoster virus (VZV) using a self-assembled protein microarray. Mol Biosyst 6, (9), 1604-10.
12. Montor, W. R.; Huang, J.; Hu, Y.; Hainsworth, E.; Lynch, S.; Kronish, J. W.; Ordonez, C. L.; Logvinenko, T.; Lory, S.; LaBaer, J., Genome-wide study of Pseudomonas aeruginosa outer membrane protein immunogenicity using self-assembling protein microarrays. Infect Immun 2009, 77, (11), 4877-86.
13. Jackman, R. J.; Duffy, D. C.; Ostuni, E.; Willmore, N. D.; Whitesides, G. M., Fabricating large arrays of microwells with arbitrary dimensions and filling them using discontinuous dewetting. Analytical Chemistry 1998, 70, (11), 2280-2287.
14. Moerman, R.; van Dedem, G. W. K., A coverslip method for controlled parallel sample introduction into arrays of (sub)nanoliter wells for quantitative analysis. Analytical Chemistry 2003, 75, (16), 4132-4138.
15. Liu, H. B.; Ramalingam, N.; Jiang, Y.; Dai, C. C.; Hui, K. M.; Gong, H. Q., Rapid distribution of a liquid column into a matrix of nanoliter wells for parallel real-time quantitative PCR. Sensors and Actuators B-Chemical 2009, 135, (2), 671-677.
16. Newman, J. R. S.; Keating, A. E., Comprehensive identification of human bZIP interactions with coiled-coil arrays. Science 2003, 300, (5628), 2097-2101.
17. Manz, A.; Harrison, D. J.; Verpoorte, E. M. J.; Fettinger, J. C.; Paulus, A.; Ludi, H.; Widmer, H. M., Planar Chips Technology for Miniaturization and Integration of Separation Techniques into Monitoring Systems - Capillary Electrophoresis on a Chip. Journal of Chromatography 1992, 593, (1-2), 253-258.
18. Harrison, D. J.; Fluri, K.; Seiler, K.; Fan, Z. H.; Effenhauser, C. S.; Manz, A., Micromachining a Miniaturized Capillary Electrophoresis-Based Chemical-Analysis System on a Chip. Science 1993, 261, (5123), 895-897.
19. Fujii, T., Microreactor for bio-chemical reaction. Petrotech (Tokyo) 2000, 23, (11), 932-938.
20. Takahiko, N.; Teruo, F., Parallel Cell-free Protein Synthesis on an Integrated Microchip Monthly journal of the Institute of Industrial Science, University of Tokyo 2001, 53, (2), 96-99.
21. Yamamoto, T.; Hino, M.; Kakuhata, R.; Nojima, T.; Shinohara, Y.; Baba, Y.; Fujii, T., Evaluation of cell-free protein synthesis using PDMS-based microreactor Arrays. Analytical Sciences 2008, 24, (2), 243- 246.
22. Fan, J. B.; Gunderson, K. L.; Bibikova, M.; Yeakley, J. M.; Chen, J.; Garcia, E. W.; Lebruska, L. L.; Laurent, M.; Shen, R.; Barker, D., Illumina universal bead arrays. In DNA Microarrays Part A: Array Platforms and Wet Bench Protocols, 2006; Vol. 410, pp 57-+.
23. LaFratta, C. N.; Walt, D. R., Very high density sensing arrays. Chemical Reviews 2008, 108, (2), 614-637.
24. Lim, C. T.; Zhang, Y., Bead-based microfluidic immunoassays: The next generation. Biosensors & Bioelectronics 2007, 22, (7), 1197-1204.
25. Zhu, H.; Klemic, J. F.; Chang, S.; Bertone, P.; Casamayor, A.; Klemic, K. G.; Smith, D.; Gerstein, M.; Reed, M. A.; Snyder, M., Analysis of yeast protein kinases using protein chips. Nature Genetics 2000, 26, (3), 283-289.
26. Angenendt, P.; Nyarsik, L.; Szaflarski, W.; Glokler, J.; Nierhaus, K. H.; Lehrach, H.; Cahill, D. J.; Lueking, A., Cell-free protein expression and functional assay in nanowell chip format. Analytical Chemistry 2004, 76, (7), 1844-1849.
27. Kinpara, T.; Mizuno, R.; Murakami, Y.; Kobayashi, M.; Yamaura, S.; Hasan, Q.; Morita, Y.; Nakano, H.; Yamane, T.; Tamiya, E., A picoliter chamber array for cell-free protein synthesis. Journal of Biochemistry 2004, 136, (2), 149-154.
28. Yunker, P. J.; Still, T.; Lohr, M. A.; Yodh, A. G., Suppression of the coffee-ring effect by shape-dependent capillary interactions. Nature 2011, 476, (7360), 308-11.
29. Schwartz, B.; Robbins, H., Chemical Etching of Silicon .4. Etching Technology. Journal of the Electrochemical Society 1976, 123, (12), 1903-1909.
30. Qiu, J.; LaBaer, J., Nucleic acid programmable protein array a just-in-time multiplexed protein expression and purification platform. Methods Enzymol 2011, 500, 151-63.

The present invention is illustrated by way of the foregoing description and examples. The foregoing description is intended as a non-limiting illustration, since many variations will become apparent to those skilled in the art in view thereof. It is intended that all such variations within the scope and spirit of the appended claims be embraced thereby. Each referenced document herein is incorporated by reference in its entirety for all purposes. Changes can be made in the composition, operation and arrangement of the method of the present invention described herein without departing from the concept and scope of the invention as defined herein.
**The invention also pertains to the following:**
1. A biomolecule array, comprising
   (a) a first substrate; and
   (b) biomolecules comprising at least 10 isolated coding nucleic acids and/or at least 10 isolated polypeptides, wherein each nucleic acid and/or polypeptide is physically confined at a discrete location on the first substrate, and wherein each nucleic acid is capable of expressing its encoded product in situ at its discrete location on the substrate, and/or wherein each polypeptide is capable of a characteristic activity in situ at its discrete location on the substrate; wherein the discrete locations are separated from each other on the first substrate by a center to center spacing of between about 20 nm and about 1 mm.
2. The biomolecule array of item 1, wherein the discrete locations are separated from each other on the first substrate by a center to center spacing of between about 20 nm and about 100 µm.
3. The biomolecule array of item 1 or 2, wherein each physically confined discrete location comprises a well.
4. The biomolecule array of item 3, wherein each well has a diameter of between about 14 nm and about 0.75 mm.
5. The biomolecule array of item 3 or 4, wherein the wells are present on the first substrate at a density of between about 250 billion wells per square centimeter and about 100 well per square centimeter.
6. The biomolecule array of any of items 1-5, wherein discrete locations are functionalized.
7. The biomolecule array of any one of items 1-6, wherein each discrete location further comprises reagents for expressing the encoded nucleic acid product and/or for testing polypeptide activity.
8. The biomolecule array of any one of items 1-6, wherein each discrete location further comprises reagents for expressing the encoded nucleic acid product and/or for testing polypeptide activity and where those reagents are added to the array separately from the nucleic acids or polypeptides.
9. The biomolecule array of item 8, wherein the reagent comprises a reticulocyte lysate.
10. The biomolecule array of item 9, wherein the biomolecules comprise at least 100 isolated coding nucleic acids.
11. The biomolecule array of any one of items 1-10, wherein the biomolecules comprise at least 10 isolated polypeptides.
12. The biomolecule array of any one of items 1-10, wherein the biomolecules comprise at least 100 isolated polypeptides.
13. The biomolecule array of any one of items 1-13, wherein the nucleic acids and/or polypeptides are bound to the substrate.
14. The biomolecule array of any one of items 1-14, wherein either (i) the array further comprises a capture substrate adapted to mate with the first substrate wherein the capture substrate is functionalized to capture and isolate the expression product; or (ii) the discrete locations are further functionalized to capture and isolate the expression product.
15. An array comprising
   (a) a silicon-containing substrate, having a surface comprising a plurality of nanowells at discrete locations wherein the discrete locations are separated from each other on the silicon-containing substrate by a center to center spacing of between about 20 nm and about 1 mm; and
   (b) biomolecules capable of capturing another molecule located at each discrete location.
16. The array of item 15, wherein the discrete locations are separated from each other on the first substrate by a center to center spacing of between about 20 nm and about 100 µm.
17. The array of item 15 or 16, wherein the biomolecules comprise antigen or antibodies, and the like.
18. The array of item 17, wherein the biomolecules are attached to the surface of the first substrate through a divalent linking group to a functional group capable of binding to or associating with the surface of the substrate.
19. The array of any one of items 15 - 18, wherein the silicon-containing substrate is a silica or silicon substrate.
20. The array of item 19, wherein the substrate comprises a glass or a silicon wafer.
21. The array of any one of items 1-20, wherein the wells are sealed.
22. The array of item 21 wherein the wells are sealed by a mating substrate.
23. A method for in situ nucleic acid expression, comprising
   (a) contacting each discrete location of the array of any one of items 1-22 with reagents for nucleic acid expression to form a mixture; and
   (b) incubating the mixture under conditions suitable for nucleic acid expression.
24. A method for expressing and capturing a product, comprising
   (a) contacting a nucleic acid array with reagents for nucleic acid expression to form a mixture, wherein the nucleic acid array comprises a first substrate and at least 10 isolated coding nucleic acids physically confined at a discrete location on a first substrate, and wherein each nucleic acid is capable of expressing its encoded product in situ at its discrete location on the first substrate;
   (b) optionally, bringing the first substrate into the proximity of a capture substrate;
   (c) incubating the mixture under conditions suitable for production of nucleic acid expression products; and
   (d) capturing and isolating the expression products on the first substrate or, when present, the capture substrate.
25. The method of item 24, wherein the capture substrate comprises a second substrate that comprises a plurality of physically confined discrete locations that match the physically confined discrete locations on the first substrate.
26. The method of item 24 or 25, wherein the method comprises precoating the first substrate or the capture substrate, when present, with a first member of a binding pair, wherein the expression products comprise a second member of the binding pair, and wherein the incubating is done under conditions suitable for binding of the first member and the second member of the binding pair, resulting in capture of the expression product on the precoated substrate.
27. The method of item 26, wherein the method comprises precoating the first substrate with a first member of a binding pair.
28. The method of item 26, wherein the method comprises precoating the capture substrate with a first member of a binding pair
29. The method of any one of items 24-28, wherein the capture substrate is contacted to the first substrate to form a seal.
30. The method of any one of items 23-29, wherein each discrete location comprises a well.
31. The method of any one of items 23-30, wherein the nucleic acid expression comprises RNA expression.
32. The method of any one of items 23-31, wherein the nucleic acid expression comprises polypeptide expression.
33. The method of item 32, wherein the reagents comprise an in vitro expression system.
34. The method of item 32, wherein the method further comprises modifying the polypeptide capture on the capture substrate.
35. The method of any one of items 32-34, further comprising stabilizing the captured polypeptides on the capture substrate to limit protein diffusion.
36. The method of item 35, wherein the stabilizing comprises freezing the captured polypeptides on the capture substrate.

## Claims

1. A biomolecule array, comprising:
(a) a first substrate comprising wells or tubes;
(b) biomolecules comprising at least 10 isolated coding nucleic acids, wherein each nucleic acid is physically confined at a discrete location on the first substrate defined by one of the wells or one of the tubes, and wherein each nucleic acid is capable of expressing an encoded protein appended with an epitope tag in situ at its discrete location on the substrate; and
(c) reagents for expressing proteins with the epitope tag within each well or each tube;
wherein the discrete locations are separated from each other on the first substrate by a center to center spacing of between about 20 nm and about 1 mm,
wherein the discrete locations are further functionalized to capture and isolate the expressed protein with the epitope tag.

2. The biomolecule array of claim 1, wherein:
(i) the well diameter is between 10% and 95% of the spacing,
(ii) the well depth is between 10% and 300% of the well diameter, and
(iii) the well density is 1/(spacing)2 and 1/(spacing x (0.75)0.5)2.

3. The biomolecule array of claim 1 or 2, wherein the discrete locations are separated from each other on the first substrate by a center to center spacing of between about 20 nm and about 100 µm.

4. The biomolecule array of any one of claims 1 to 3, wherein the reagent for expressing proteins comprises cell lysate.

5. The biomolecule array of any one of claims 1 to 4, wherein the functionalized discrete locations comprise an antibody for the expression product, or a halotag ligand if the expressed protein comprises a halotag.

6. The biomolecule array of any one of claims 1 to 5, wherein each well has a diameter of between about 14 nm and about 0.75 mm.

7. The biomolecule array of any of claims 1 to 6, wherein the wells are present on the first substrate at a density of between about 250 billion wells per square centimeter and about 100 wells per square centimeter.

8. The biomolecule array of any one of claims 1 to 7, comprising captured proteins for use in one or more processes selected from the group consisting of surface plasmon resonance (SPR), MALDI mass spectrometry, and post-translational modification.

9. The biomolecule array of any one of claims 1 to 8, wherein the first substrate contains silicon.

10. The biomolecule array of any one of claims 1 to 9, wherein the surface of each discrete location is further functionalized to be hydrophobic or hydrophilic.

11. The biomolecule array of any one of claims 1 to 10, further comprising a lip around each well, a silicone-rubber gasket around each well, or a mating substrate that forms a seal around each well of the substrate.

12. The biomolecule array of any one of claims 1 to 11, further comprising a microsieve membrane for covering the array.

13. A method for detecting biomolecules on the biomolecule array of any one of claims 1 to 12, the method comprising the steps of:
(a) scanning the array at various depths; and
(b) combining the intensity reading at each pixel of the resulting scanned images.

14. A method for in situ nucleic acid expression, the method comprising the steps of:
(a) contacting each discrete location of the biomolecule array of any one of claims 1 to 12 with reagents for nucleic acid expression to form a mixture; and
(b) incubating the mixture under conditions suitable for nucleic acid expression.

15. A method for expressing and capturing an expression product, the method comprising the steps of:
(a) contacting each discrete location of the biomolecule array of any one of claims 1 to 12 with reagents for nucleic acid expression to form a mixture;
(b) incubating the mixture under conditions suitable for nucleic acid expression; and
(c) capturing and isolating the expression products on the first substrate.
